# EUROPEAN PATENT APPLICATION

(11) **EP 4 647 015 A2**
(43) Date of publication of application: **12.11.2025**
(21) Application number: 25199235.0
(22) Date of filing: 13.07.2020
(51) Int. Cl.: A61B 17/04

(54) **DEVICE ALLOWING LARGE BORE TRANSSEPTAL ACCESS WITH SUBSEQUENT ATRIAL RE-ACCESS**

(30) Priority: 08.06.2020 US 202063036435 P
(62) Divisional of application: 20841488.8
(71) Applicant: AMX Technologies, LLC, Plymouth, Minnesota 55441 (US)
(72) Inventor: SARABIA, Jaime E., Mableton, GA 30126 (US); FARRELL, Richard, White Bear Lake, MN 55110 (US); COYLE, Daniel P., St. Louis Park, MN 55416 (US)
(74) Representative: KIPA AB

(57) **Abstract**

The present disclosure relates to medical devices. More particularly, this disclosure describes a vascular device allowing large bore transseptal access with subsequent atrial re-access by preplacing closures/tissue approximating sutures prior to creating a septostomy. Generally, the device may include a delivery catheter for puncturing and cutting the interatrial septum. An anchor of the delivery catheter may secure the suture in an atrium to a septum wall, for example, the left atrium. Incisions may be made by an expandable cutting implement which may use mechanical or radio frequency (RF) energy without interfering with the suture. A therapeutic instrument may be advanced through the tissue plane after the incisions are made by the cutting implement. Closure of the incision may be performed with the previously placed sutures.

## Description

### RELATED DISCLOSURE

This disclosure claims priority to U.S. Provisional Application Serial No. 63/036,435 filed June 8, 2020 titled *Large Bore Septal Closure* and U.S. Provisional Application Serial No. 62/873,383 filed July 12, 2019 titled *Large Bore Atrial Preclose,* both of which are hereby referred to.

### TECHNICAL FIELD

The present disclosure relates to medical devices and more particularly, to transcatheter-delivered interatrial septal crossing and closing techniques for a large bore instrument allowing access into an atrium (e.g., the left atrium) with subsequent atrial re-access.

### BACKGROUND

An increasingly common approach for left heart catheter procedures may be to puncture and cross an interatrial septum using a mechanical or radio frequency (RF) powered needle. This procedure is generally straightforward for small bore catheters, which are typically less than 24 French. If larger catheters or bores, however, are to be sent across the atrial septum, a puncture site dilation is typically used in order to advance the catheter through the septum. Current methodologies for dilating the initial septal puncture site may involve the use of a dilator, or by inflating a balloon, to open the access site. This may use multiple tool exchanges by the physician and may have undesirable consequences on the tissue due to the uncontrolled nature of the dilation techniques.

In addition, minimally-invasive, catheter-based therapies are being developed that allow physicians to provide treatments to patients whose existing comorbidities may preclude them from having a needed, but more invasive, surgical procedure. Over the last few years, catheter based procedures have developed which may involve implantation of repair or replacement mitral valves, which may use large bore transseptal access. The transseptal puncture may result in the formation of an iatrogenic atrial septal defect which may need to be subsequently closed by an atrial septal defect device. However, that atrial septal defect device may preclude, or make difficult, subsequent transseptal crossing.

The present disclosure provides for a device allowing large bore transseptal access with subsequent atrial re-access and method thereof that addresses the above identified concerns. A controlled and precise atrial septostomy that permits passage of the large bore device across the interatrial septum and then provides a rapid and permissive closure of the procedurally created atrial septal defect is described herein. The word permissive may be defined as a mechanism which the septal defect is closed and may allow future crossings of the interatrial septum by standard transseptal methods. Other benefits and advantages will become clear from the disclosure provided herein and those advantages provided are for illustration. The statements in this section merely provide the background related to the present disclosure and does not constitute prior art.

### SUMMARY

This summary is provided to introduce a selection of concepts in a simplified form that are further described below in the DESCRIPTION OF THE DISLCOSURE. This summary is not intended to identify key features of the claimed subject matter, nor is it intended to be used as an aid in determining the scope of the claimed subject matter. The present invention is defined by the appended claims only, in particular independent claim 1. References to "embodiments" throughout the description which are not under the scope of the appended claims merely represents possible example executions.

According to one aspect of the present disclosure, a vascular device for performing a transseptal puncture is provided. The device may include a body, an anchor extending from a distal end of the body through a shaft disposed within the body, at least one suture coupled to at least one needle within the anchor, at least one catch extending from the body to pull the at least one needle into the body for placing the at least one suture, and a cutting implement between the body and anchor coupled to an actuating shaft aligned with the at least one suture.

According to another aspect of the present disclosure, a septal orifice closure apparatus allowing re-access is provided. The apparatus may include a body on a first side of a septal orifice in a septum of a heart, an anchor on a second side of the septal orifice extending from a distal end of the body through a shaft disposed within the body, at least one suture coupled to at least one needle disposed within the anchor, at least one catch extending from the body to pull the at least one needle into the body for placing the at least one suture, and a cutting implement between the body and anchor coupled to an actuating shaft aligned with the at least one suture.

According to yet another aspect of the present disclosure, a vascular closure apparatus is provided. The apparatus may include an anchor positioned through a puncture in a vessel wall and operable between retracted and expanded positions from a body, at least one suture disposed within the anchor, at least one needle coupled to the at least one suture extending through the vessel wall adjacent to the puncture to connect the at least one suture when the anchor is in the expanded position, at least one catch extending from the body to pull the at least one needle into the body for placing the at least one suture, and a cutting implement between the body and anchor coupled to an actuating shaft aligned with the at least one suture.

According to another aspect of the present disclosure, a method of performing a septal crossing in a vessel wall is provided. The method may include providing a delivery catheter having a body and an anchor, inserting the anchor through a puncture in the vessel wall, operating the anchor into an expanded position capturing the vessel wall between the body and the anchor to expose at least one needle, capturing the at least one needle through the vessel wall adjacent to the puncture and into engagement with at least one suture, and positioning the at least one suture in the vessel wall.

According to one aspect of the present disclosure, a vascular apparatus is provided. The apparatus may include a delivery system having at least one anchor penetrating a tissue plane, the at least one anchor having a suture, a cutting implement positioned into the tissue plane facilitating an incision, a therapeutic instrument advanced into the incision, and a fastener securing the suture with tissue of the tissue plane.

According to yet another aspect of the present disclosure, a septal orifice closure apparatus is provided. The apparatus may include a first pledget introduced into a tissue plane through a cannula, wherein the first pledget is coupled to a control line tensioning the first pledget after introduction into the tissue plane, a second pledget introduced into the tissue plane through the cannula, wherein the second pledget is coupled to a control line tensioning the second pledget after introduction into the tissue plane, a cutting implement making an incision between the first pledget and second pledget, a therapy device passed through the incision, and a knot made of the control line of the first pledget and the control line of second pledget tensioning the first pledget and second pledget with tissue from the tissue plane therebetween.

According to another aspect of the present disclosure, a device for puncturing an atrial septum of a patient is provided. The device may include a body, a tip extending from a distal end of the body, and a cutting member in a collapsed state disposed between the body and tip, wherein the tip followed by the cutting member penetrates into a tissue plane, the cutting member expanded after passing through the tissue plane.

According to one aspect of the present disclosure, a vascular apparatus is provided. The apparatus may include a delivery system, a tip extending from a distal end of the delivery system, and a cutting implement disposed between the delivery system and tip.

According to another aspect of the present disclosure, a method of instrumenting the left atrium is provided. The method may include puncturing a septum with a needle, placing at least one suture behind the septum, advancing a therapeutic instrument into the puncture, and cinching the at least one suture closing the puncture.

According to another aspect of the present disclosure, a method of closing a septal orifice is provided. The method may include creating a transseptal access through a wire, inserting a delivery catheter over the wire, enlarging the transseptal access through a cutting implement of the delivery catheter, inserting at least one suture coupled to a needle that passes around the transseptal access, cinching the transseptal access with the at least one suture, and removing the delivery catheter.

### BRIEF DESCRIPTION OF THE DRAWINGS

The novel features believed to be characteristic of the disclosure are set forth in the appended claims. In the descriptions that follow, like parts are marked throughout the specification and drawings with the same numerals, respectively. The drawing FIGURES are not necessarily drawn to scale and certain FIGURES may be shown in exaggerated or generalized form in the interest of clarity and conciseness. The disclosure itself, however, as well as a preferred mode of use, further objectives and advantages thereof, will be best understood by reference to the following detailed description of illustrative embodiments when read in conjunction with the accompanying drawings, wherein:
**FIG. 1** is a front view schematic representation of an illustrative human venous circulatory system of a patient with a guidewire routed from the femoral vein into the right atrium in accordance with one aspect of the present disclosure;
**FIG. 2** is a front view schematic representation of the illustrative human venous circulatory system of the patient with an exemplary vascular device advanced into a right atrium in accordance with one aspect of the present disclosure;
**FIG. 3** is a cross-sectional illustration of a heart with the exemplary vascular device positioned at an atrial septum and a septal penetrator advanced across the atrial septum into a left atrium in accordance with one aspect of the present disclosure;
**FIG. 4** is a cross-sectional illustration of the heart with the exemplary vascular device advanced into the left atrium across the atrial septum and the septal penetrator withdrawn in accordance with one aspect of the present disclosure;
**FIG. 5** is a cross-sectional illustration of the heart showing illustrative initial incisions followed by lengthening them in accordance with one aspect of the present disclosure;
**FIG. 6** is an illustrative side view of the exemplary vascular device in accordance with one aspect of the present disclosure;
**FIG. 7** is an isometric view of a distal end of the exemplary vascular device in a low profile condition in accordance with one aspect of the present disclosure;
**FIG. 8** is an isometric view of the distal end of the exemplary vascular device viewed from a different perspective with a portion of the device advanced revealing components used to puncture tissue and pass sutures in accordance with one aspect of the present disclosure;
**FIG. 9** is an isometric view of the distal end of the exemplary vascular device with the portion of the device advanced through the tissue of the atrial septum in accordance with one aspect of the present disclosure;
**FIG. 10** is a cross sectional view of the exemplary vascular device having an illustrative lumen configuration in accordance with one aspect of the present disclosure;
**FIG. 11** is an isometric, sectioned view of the distal end of the exemplary vascular device revealing the inner geometry of components therein in accordance with one aspect of the present disclosure;
**FIG. 12** is an isometric view of the distal end of the exemplary vascular device revealing snares that have been advanced out of a body of the vascular device in accordance with one aspect of the present disclosure;
**FIG. 13** is an isometric view of the distal end of the exemplary vascular device after four suture needles and suture ends have been passed through the tissue, snared and pulled through the length of the device in accordance with one aspect of the present disclosure;
**FIG. 14** is an isometric view of the distal end of the exemplary vascular device after the suture has been pulled completely through the length of the device and is now pulled taught against the tissue in accordance with one aspect of the present disclosure;
**FIG. 15** is an isometric view of the distal end of the vascular device after a cutting implement has been partially advanced revealing the cutting elements in accordance with one aspect of the present disclosure;
**FIG. 16** is an isometric view of the distal end of the vascular device with the cutting implement expanded in accordance with one aspect of the present disclosure;
**FIG. 17****.** is an isometric view of the exemplary vascular device positioned to cut the tissue in accordance with one aspect of the present disclosure;
**FIG. 18** is an isometric view of sutures that would be inserted into the tissue in accordance with one aspect of the present disclosure;
**FIG. 19** is an isometric view of an incision after the tissue is engaged with the suture in accordance with one aspect of the present disclosure;
**FIGS. 20A-L**are various illustrative cut patterns that may be created using the vascular device in the atrial septum which may be made from at least one cutting implement that may be rotated and used multiple times in accordance with one aspect of the present disclosure;
**FIG. 21** is an isometric view of an illustrative cut pattern for incision to promote tissue edge apposition in accordance with one aspect of the present disclosure;
**FIG. 22** is an isometric view of the illustrative cut pattern for incision to promote the tissue edge apposition, while under slight tension, demonstrating tissue edge control and overlap of edges with tension applied in accordance with one aspect of the present disclosure;
**FIG. 23** is an isometric view of the cut patterns for incision to promote the tissue edge apposition and an illustrative helical anchor controlling tissue edges in accordance with one aspect of the present disclosure;
**FIG. 24** is an isometric view of an illustrative expandable radio frequency (RF) cutting implement with four expandable members in accordance with one aspect of the present disclosure;
**FIG. 25** is an isometric view of an exemplary vascular device with the expandable cutting implement having a tip for piercing the tissue in accordance with one aspect of the present disclosure;
**FIG. 26** is an isometric view of the exemplary vascular device with the illustrative expandable cutting implement placed beyond the tissue in accordance with one aspect of the present disclosure;
**FIG. 27** is an isometric view of the exemplary vascular device with the illustrative expandable cutting implement making incisions into the tissue in accordance with one aspect of the present disclosure;
**FIG. 28** is an isometric view of the exemplary vascular device with the illustrative expandable cutting implement advancing anchor mechanisms in accordance with one aspect of the present disclosure;
**FIG. 29** is an isometric view of the exemplary vascular device with the illustrative expandable cutting implement with push anchors further inserted to advance the anchor mechanisms in accordance with one aspect of the present disclosure;
**FIG. 30** is an isometric view of the exemplary vascular device having the anchor mechanisms removed in accordance with one aspect of the present disclosure;
**FIG. 31** is an isometric view of the exemplary cutting implement removed from the tissue leaving tissue anchors against it in accordance with one aspect of the present disclosure;
**FIG. 32** is an isometric view of an exemplary toggle within the tissue in accordance with one aspect of the present disclosure;
**FIG. 33** is an isometric view of an illustrative cutting implement having an atraumatic tip deployed from an exemplary vascular device in accordance with one aspect of the present disclosure;
**FIG. 34** is an isometric view of an illustrative cutting implement having a slit in a sheath deployed from an exemplary vascular device in accordance with one aspect of the present disclosure;
**FIG. 35** is a side view of the illustrative cutting implement having a cutting element extending from the slit in the sheath in accordance with one aspect of the present disclosure;
**FIG. 36** is an isometric view of a distal end of the exemplary cutting implement in accordance with one aspect of the present disclosure;
**FIG. 37** is a cross-sectional illustration of the cutting implement in accordance with one aspect of the present disclosure;
**FIGS. 38A-E** are schematics showing how the exemplary cutting implements may be used to optimize cutting performance and minimize power input in accordance with one aspect of the present disclosure;
**FIG. 39** is an isometric view of exemplary tissue being crossed using an illustrative guidewire in accordance with one aspect of the present disclosure;
**FIG. 40** is an isometric view of suture anchors in delivery sheaths in accordance with one aspect of the present disclosure;
**FIG. 41** is an isometric view of illustrative suture anchors in delivery sheaths about to penetrate the tissue in accordance with one aspect of the present disclosure;
**FIG. 42** is an isometric view of the illustrative suture anchors in delivery sheaths engaging or penetrating the tissue in accordance with one aspect of the present disclosure;
**FIG. 43** is an isometric view of the illustrative suture anchors in delivery sheaths engaging the tissue with suture control lines attached in accordance with one aspect of the present disclosure;
**FIG. 44** is an isometric view of an illustrative cutting implement in a sheathed position in accordance with one aspect of the present disclosure;
**FIG. 45** is an isometric view of the illustrative cutting implement in an unsheathed position in accordance with one aspect of the present disclosure;
**FIG. 46** is an isometric view of the illustrative cutting implement in an unsheathed position and expanded in accordance with one aspect of the present disclosure;
**FIG. 47** is an isometric view of the illustrative cutting implement making an incision or cut into the tissue in accordance with one aspect of the present disclosure;
**FIG. 48** is an isometric view of an illustrative incision or cut in the tissue with a guidewire passing through it in accordance with one aspect of the present disclosure;
**FIG. 49** is an isometric view of an advancing of an exemplary therapeutic instrument passing through the cut in the tissue over the guidewire in accordance with one aspect of the present disclosure;
**FIG. 50** is an isometric view of an illustrative tissue anchor lock with helical barbs in accordance with one aspect of the present disclosure;
**FIG. 51** is an isometric view of the illustrative tissue anchor lock with helical barbs engaged in the tissue passed over the suture control lines in accordance with one aspect of the present disclosure;
**FIG. 52** is an isometric view of the illustrative tissue anchor lock with helical barbs engaged in the tissue passed over the suture control lines and the control lines trimmed to the level of the anchor in accordance with one aspect of the present disclosure;
**FIG. 53** is an isometric view of the illustrative tissue anchor lock with helical barbs engaged in an other side of the tissue in accordance with one aspect of the present disclosure;
**FIG. 54** is an isometric view of an illustrative pledget made from biocompatible or bio-absorbable material in accordance with one aspect of the present disclosure;
**FIG. 55** is an isometric view of an exemplary cannula piecing heart tissue in accordance with one aspect of the present disclosure;
**FIG. 56** is an isometric view of the exemplary cannula piecing heart tissue and the illustrative pledget made from biocompatible or bioabsorbable material advanced through the cannula in accordance with one aspect of the present disclosure;
**FIG. 57** is an isometric view of the exemplary cannula piecing heart tissue and the illustrative pledget made from biocompatible or bioabsorbable material advanced out of the cannula in accordance with one aspect of the present disclosure;
**FIG. 58** is an isometric view of the exemplary cannula piecing heart tissue and the illustrative pledget made from biocompatible or bioabsorbable material advanced out of the cannula and a control line tensioned to shorten the pledget in accordance with one aspect of the present disclosure;
**FIG. 59** is an isometric view of the illustrative pledget made from biocompatible or bioabsorbable material tensioned to shorten the pledget with the exemplary cannula piecing heart tissue withdrawn and retained on a heart tissue surface in accordance with one aspect of the present disclosure;
**FIG. 60** is an isometric view of an illustrative concentric pledget made from biocompatible or bioabsorbable material in accordance with one aspect of the present disclosure;
**FIG. 61** is an isometric view of the exemplary cannula piecing heart tissue next to the pledget in accordance with one aspect of the present disclosure;
**FIG. 62** is an isometric view of the exemplary cannula piecing heart tissue and the illustrative concentric pledget made from biocompatible or bioabsorbable material advanced out of the cannula in accordance with one aspect of the present disclosure;
**FIG. 63** is an isometric view of the exemplary cannula piecing heart tissue and the illustrative concentric pledget made from biocompatible, or bioabsorbable material advanced out of the cannula and a control tensioned to shorten the pledget in accordance with one aspect of the present disclosure;
**FIG. 64** is an isometric view of the illustrative concentric pledget made from biocompatible or bioabsorbable material tensioned to shorten the pledget and an exemplary incision made between the pledgets in accordance with one aspect of the present disclosure;
**FIG. 65** is an isometric view of an exemplary therapeutic instrument placed into the incision between the pledgets into the tissue in accordance with one aspect of the present disclosure;
**FIG. 66** is an isometric view of an illustrative knot advanced up to a heart tissue with the two pledget control lines in accordance with one aspect of the present disclosure;
**FIG. 67** is an isometric view of the illustrative knot advanced up to the heart tissue with the two pledget control lines and tightened from a knot side of the tissue in accordance with one aspect of the present disclosure;
**FIG. 68** is an isometric view of the illustrative knot advanced up to the tissue with the two pledget control lines tightened from the pledget side in accordance with one aspect of the present disclosure;
**FIG. 69** is an isometric view of the illustrative incision closed between the pledgets in accordance with one aspect of the present disclosure; and
**FIG. 70** is an illustrative flow chart showing exemplary processes for allowing a large bore transseptal access with subsequent atrial re-access in accordance with one aspect of the present disclosure.

### DESCRIPTION OF THE DISCLOSURE

The description set forth below in connection with the appended drawings is intended as a description of exemplary embodiments of the disclosure and is not intended to represent the only forms in which the present disclosure may be constructed and/or utilized. The description sets forth the functions and the sequence of blocks for constructing and operating the disclosure in connection with the illustrated embodiments. It is to be understood, however, that the same or equivalent functions and sequences may be accomplished by different embodiments that are also intended to be encompassed within the spirit and scope of this disclosure.

The present disclosure relates to medical devices. More particularly, this disclosure describes a vascular device allowing large bore transseptal access with subsequent atrial re-access by preplacing closures/tissue approximating sutures prior to creating a septostomy. Generally, the device may include a delivery catheter for puncturing and cutting the interatrial septum. An anchor of the delivery catheter may secure the suture in an atrium to a septum wall, for example, the left atrium. Incisions may be made by an expandable cutting implement which may use mechanical or radio frequency (RF) energy without interfering with the suture. The suture may be made of a high temperature resistant material to prevent damage if it is in contact with the cutting implement. A therapeutic instrument may be advanced through the tissue plane after the incisions are made by the cutting implement. Closure of the incision may be performed with the previously placed sutures.

Numerous other modifications or configurations to the vascular device will become apparent from the description provided below. For example, closing the incision of the interatrial septum may involve needles that may puncture the septum and pass push anchors into the tissue. Control lines tied to two or more pledgets may be also used and placed through the tissue plane via a cannula to promote tissue edge overlap and apposition.

Advantageously, the initial puncture with suture management nearby allows for a rapid closure of the procedurally created iatrogenic atrial septal defect (ASD) while permissively allowing multiple instruments within the single delivery catheter access at the atrial septum. Incisions made therefrom are easily cinched allowing re-access through the anchors/sutures. The vascular device may be useful in procedures requiring large bore trans-venous access to a left atrium for transcatheter mitral valve replacement, where the delivery systems commonly create a large residual ASD. Other benefits and advantages will become clear from the disclosure provided herein and those advantages provided are for illustration.

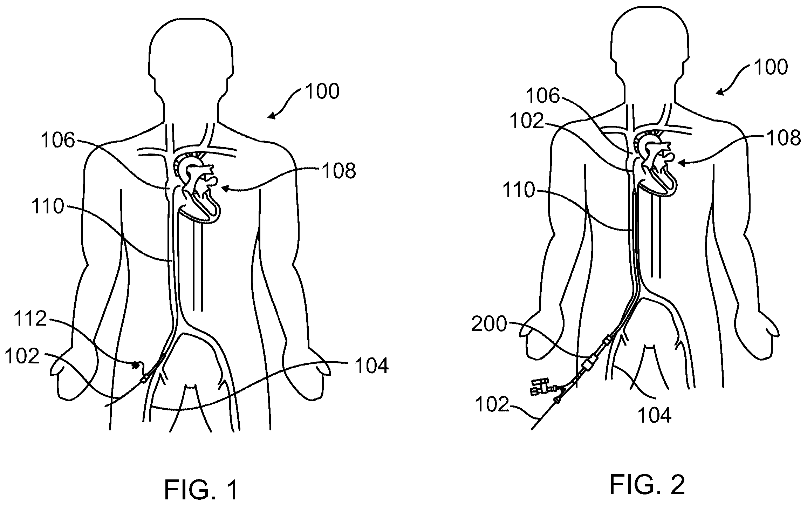

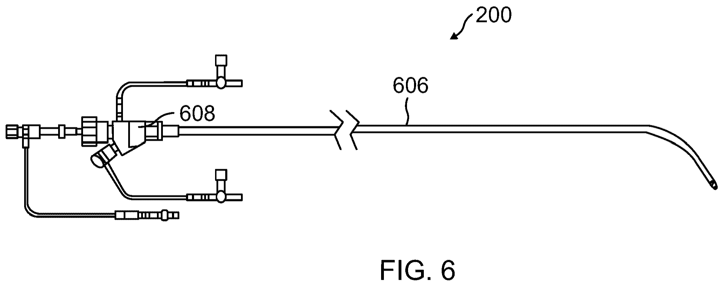

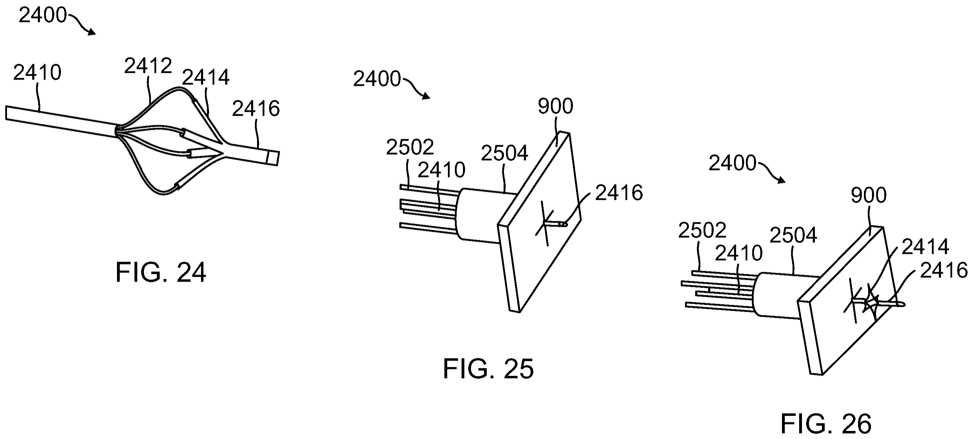

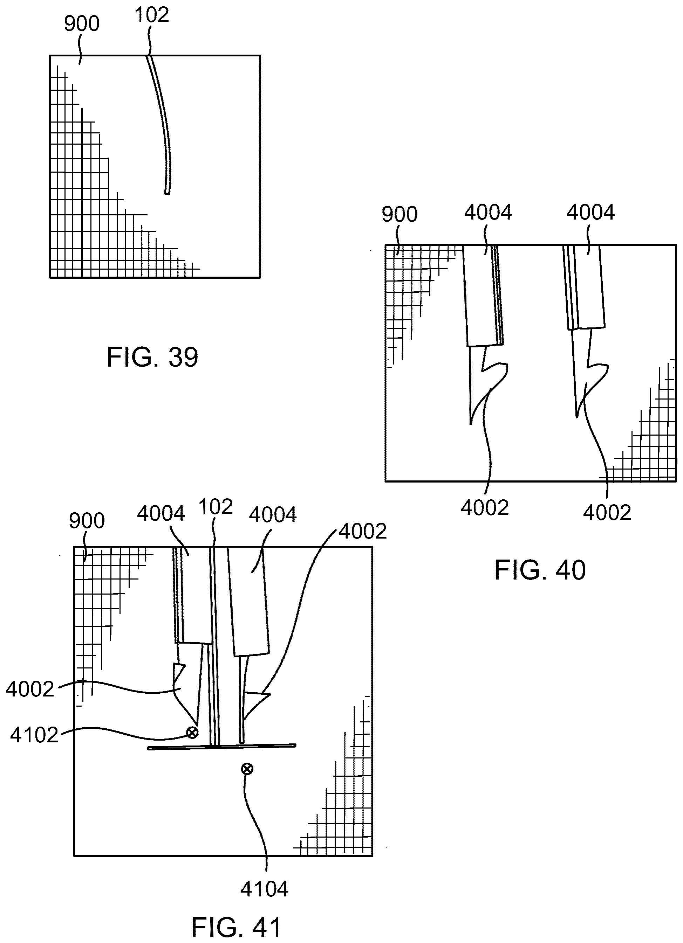

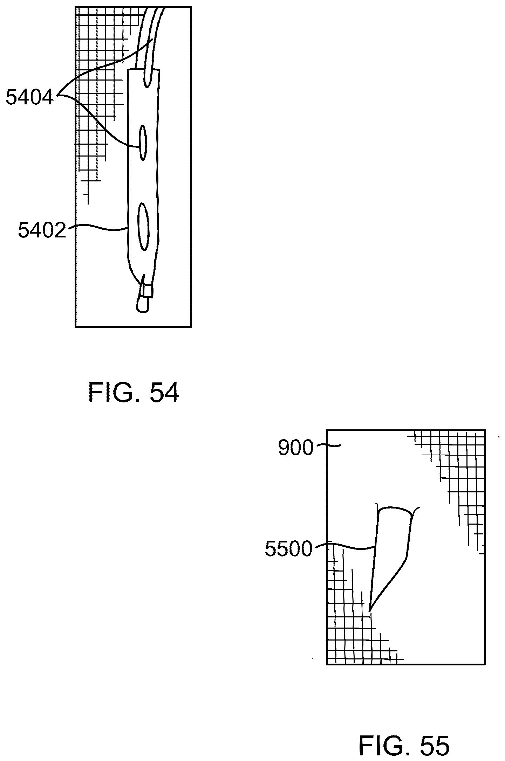

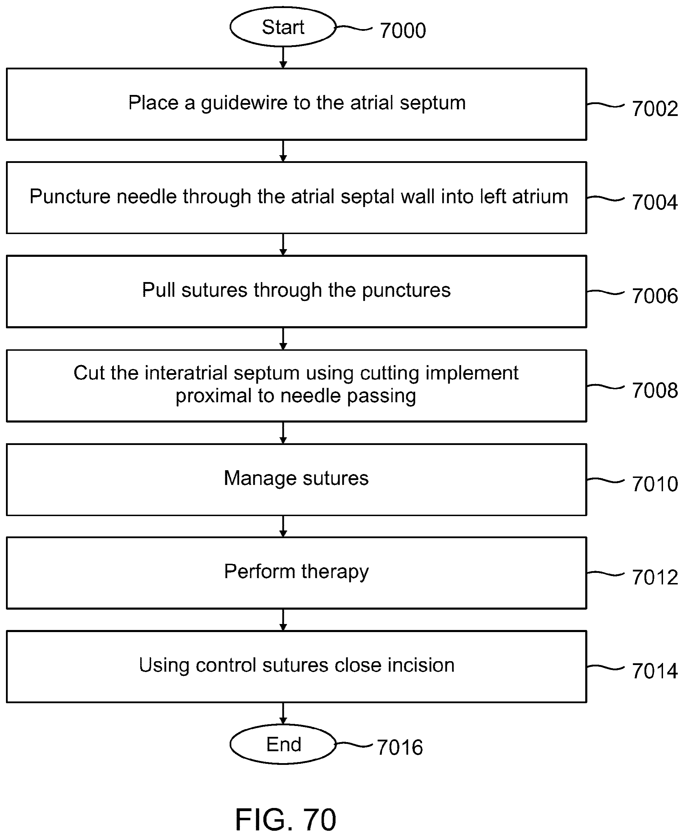

**FIGS. 1-5** represent a human venous circulatory system including a heart with an exemplary vascular device with guidewire defined therein while **FIGS. 6-23** describe a first embodiment of the device with illustrative incisions. **FIGS. 24-38** describe a second embodiment of the vascular device with additional illustrative incisions. **FIGS. 39-53** describe a third embodiment with delivery sheaths and a helical anchor for tying tissue together. **FIGS. 54-69** provide a fourth embodiment localizing a plurality of pledgets and sutures made of biodegradable materials secured together with a knot to allow re-access. **FIG. 70** provides for different techniques. Components described below within the embodiments may be interchanged, removed, or added within or to one another to come up with derivatives of the device which are within the scope of the present disclosure.

Turning to **FIG. 1**, a front view schematic representation of an illustrative human venous circulatory system of a patient **100** with a guidewire **102** routed from a femoral vein **104** into a right atrium **106** in accordance with one aspect of the present disclosure is provided. The guidewire **102** may permit a continuous presence whereby multiple tools may be exchanged. For example, these tools may provide placement of pre-closure sutures, subsequent controlled atrial septostomy by a retractable blade, and delivery of a large bore catheter or other medical device to the left atrium.

Initially, as shown, a vascular introduction sheath **112** may be inserted into the right femoral vein **104** via a percutaneous puncture or incision. Alternatively, the vascular introduction sheath **112** may be placed into a non-femoral site such as a jugular vein, subclavian artery, subclavian vein, or brachial artery and vein. Other approaches or access sites may include an approach of the opposite leg from the therapy catheter.

The guidewire **102** may be inserted through a vascular introduction sheath **112** and routed cranially up the inferior vena cava **110** to the right atrium **106,** one of the chambers of the heart **108.** In this illustration, the left anatomical side of the patient **100** is toward the right. The guidewire **102** may be placed so that it is used to direct therapeutic or diagnostic catheters into a region of the heart **108.**

The venous circulation, through which the guidewire **102** has been routed, may generally be at a lower pressure between 0 and 20 mm Hg than is the systemic circulation, of which the descending aorta is a part of. The pressure within the systemic circulation may range from 60 to over 300 mm Hg depending on the level of hypertension or hypotension existent in the patient **100.** By accessing the heart **108** through the venous circulation, at the femoral vein **104,** the chance of hemorrhage from the catheter insertion site may be minimized.

**FIG. 2** is a front view schematic representation of the illustrative human venous circulatory system of the patient **100** with an exemplary vascular device **200** advanced into a right atrium **106** in accordance with one aspect of the present disclosure. The view is a frontal illustration, looking posteriorly from the anterior side of the patient **100.** The vascular introduction sheath **112** of **FIG. 1** has been removed from the right femoral vein **104** and a vascular device **200** has been inserted into the venous circulation over the guidewire **102.** The device **200** may be routed through the inferior vena cava **110** into the right atrium **106** of the heart **108** through the same guidewire **102** used by the introduction sheath.

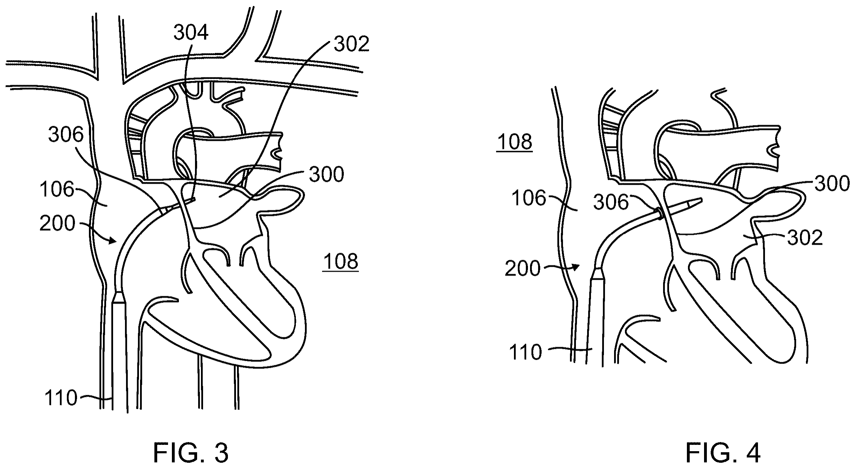

With reference to **FIG. 3****,** a cross-sectional illustration of the heart **108** with the exemplary vascular device **200** positioned at an atrial septum **300** and a septal penetrator **304** advanced across the atrial septum **300** into a left atrium **302** in accordance with one aspect of the present disclosure is shown. An ascending aorta, aortic valve, pulmonary artery, and pulmonary valve have been removed from this illustration for clarity and to show the atrial septum **300.** The body of the vascular device **200,** substantially located within the right atrium **106,** is shown with its long axis perpendicular to the atrial septum **300.** The proximal end of the vascular device **200** is shown resident within the inferior vena cava **110.** A septal penetrator **304** is shown extended through a puncture **306** in the atrial septum **300** and is routed into the left atrium **302** on a distal end of the vascular device **200.**

The septal penetrator **304** may be a needle or axially elongate structure with a sharp, pointed distal end. The septal penetrator **304** may be resident within the guidewire **102,** with the penetrator **304** being removable. The septal penetrator **304** may be actuated at the proximal end of the vascular device **200** through a control mechanism such as a button, lever, handle, or trigger which may be affixed, permanently or removably by way of a linkage, pusher rod, electrical bus, or the like that runs the length of the device **200.**

In operation, the septal penetrator **304** through a wall of the left atrium **302** opposite the atrial septum **300** may be guided and advanced using fluoroscopy, magnetic resonance imaging (MRI), ultrasound, or the like. Care may be taken not to inadvertently pierce the aorta through the penetrator **304** in the region upstream or anatomically proximal to an aortic arch of the patient **100.** The distal portion of the vascular device **200** may be bent, deflected, or articulated through an angle of between 30 and 120 degrees to achieve approximate perpendicularity with the atrial septum **300.**

The septal penetrator **304** may be solid, it may be hollow like a hypodermic needle, or it may have a "U" or "C"-shaped cross-section. The center or core of a hollow "C" or "U"-shaped septal penetrator **304** may be filled with a guidewire or other core element to prevent incorrect tissue penetration. The septal penetrator **304** may be rigid or it may be flexible but retain column strength. Such flexible configurations may include cutouts in the wall of the penetrator **304** or guidewire-like construction. The septal penetrator **304** may be initially straight or it may be initially curved. The septal penetrator **304** may be fabricated from shape memory material such as nitinol and heat treated to cause curving once the material is heated from martensitic to austenitic temperatures. Such heating may be performed using electrical heating, hot water injection, or the like. The septal penetrator may utilize energy to facilitate puncture of the septal tissue such as RF (Radio Frequency).

Referring to **FIG. 4****,** a cross-sectional illustration of the heart **108** with the exemplary vascular device **200** advanced into the left atrium **302** across the atrial septum **300** and the septal penetrator withdrawn in accordance with one aspect of the present disclosure is shown. The vascular device **200** having expanded at a distal portion has advanced across the atrial septum **300** from the right atrium **106** and into the left atrium **302** through the puncture **306.** Through this, the distal portion of the vascular device **200** may provide placement of pre-closure sutures or other apparatuses.

The proximal region, or body, of the vascular device **200** has advanced so that the proximal region is located not only in the inferior vena cava **110** but also within the right atrium **106.** This may be guided by fluoroscopy, magnetic resonance imaging (MRI), ultrasound, or the like, which was described earlier.

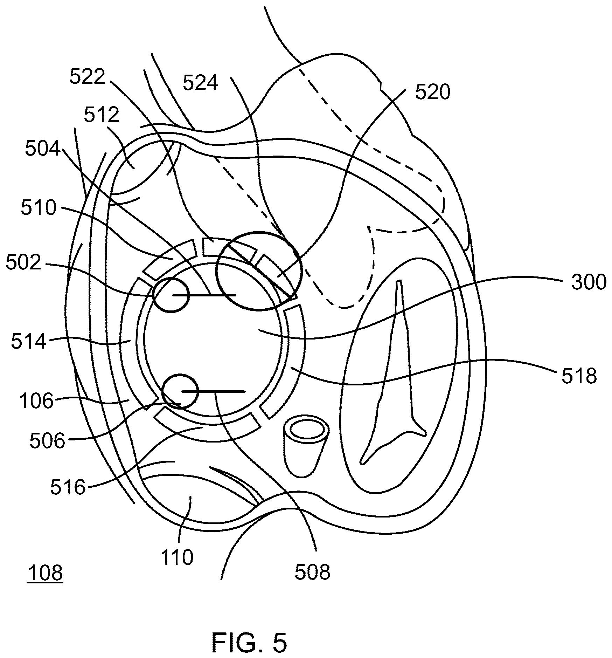

**FIG. 5** is a cross-sectional illustration of the heart **108** showing illustrative initial incisions **502** or **506** and followed by lengthening them to a user selected/controlled length in accordance with one aspect of the present disclosure. A cross-sectional view of the heart **108** is shown viewed from a right atrial side. The atrial septum **300** may be surrounded by the superior vena cava rim **510** of a superior vena cava **512,** posterior rim **514,** inferior vena cava rim **516** of the inferior vena cava **110,** atrioventricular valve rim **518,** aortic rim **520,** and superior rim **522.** The vascular device **200** may create a user defined adjustably controlled atrial septostomy by a retractable cutting implement, in conjunction with suture-mediated closure created by the atrial septostomy.

After the puncture is made into the atrial septum **300** by the septal penetrator at an ideal location, the distal end of the vascular device **200** may be inserted through the tissue plane. An initial incision **502** or **506** on the atrial septal wall may be made and then subsequently lengthened to a specified desired amount **504** or **508** appropriate to allow for a therapeutic instrument. Additionally, positional control and visualization may enable the user to avoid areas **524** of the anatomy that are not desirable to disturb such as the aortic rim **520** or superior rim **522** or puncturing outside the atrium, or cutting through myocardium

The target of the tissue incision **502 or 506** may be on the atrial septum **300** in such a location to allow access to the desired target of therapy such as the mitral valve. In one example, the incision length or size may be set to accommodate the procedural instrumentation or device without further damaging the tissue plane. The tissue when cut to a specific length that is large enough may facilitate no ripping of the tissue beyond the desired incision length. This may be accomplished by having the perimeter of the incision **502** or **506** along with the desired amount **504** or **508** match the circumference of the therapeutic instrument. In one embodiment, creating a cut that is slightly larger than the subsequent therapy catheter may enable more flexibility to the user. In a typical septal puncture, if the initial puncture site is not adequate, the user may need to retract the catheter, re-puncture, and re-dilate with the risk of tearing the tissue.

The length may also be adjusted to account for stretching of the tissue plane. The tissue rim surrounding the atrial fossa may be used or referenced to begin or limit the incision **502** or **506** of the tissue. Extending the incision **502** or **506** beyond or outside the rim of the fossa may require more force or energy to create and therefor utilized as a feedback loop to determine the location of the incision **502** or **506.**

In one example, which will be shown below, the vascular device **200** may have one cutting arm and a centering puncture member. This configuration may have the operator rotate the tool to create a slit in a desired direction. The edge of the fossa of the heart **108** may be used as the starting point as this point may be easier to puncture through first and then rotate the cutting member to cut along a direction of choice. Alternatively, two symmetrical cutting arms extending from center (with or without a centering puncture face) may be used. This configuration may allow the physician to puncture a known height or position which may allow them to be sure that the center of the cut may be at the position as they intend, as the cut is symmetric.

Turning to **FIG. 6****,** which discloses a first embodiment, an illustrative side view of the exemplary vascular device **200** in accordance with one aspect of the present disclosure is provided. The vascular device **200** may have a distal region, which may be the tip or anchor, expandable from a body of the device **200.** A length of sheath tubing **606** may extend the body to a sheath hub **608.** The tubing **606** may be substantially curved near its distal end to provide deflection of catheters in a direction approximately 180 degrees from the exit path of the guidewire **102.** As illustrated, the sheath hub **608** may be a tri port hub **608.** The hub **608** may be configured, in other embodiments, with less or more ports. Multiple instruments may be inserted and retracted from the hub **608,** which will be shown below.

Broadly described, the vascular device **200** may be used to place sutures into the atrial septum, either before or after creating a controlled atrial septostomy through instruments placed into the hub **608.** Other medical devices may gain entry and location to the left atrium, with the ability to subsequently close that ASD. Advantageously, the way the ASD is closed may permit future tissue crossings in the event that is necessary for a subsequent catheter based procedure.

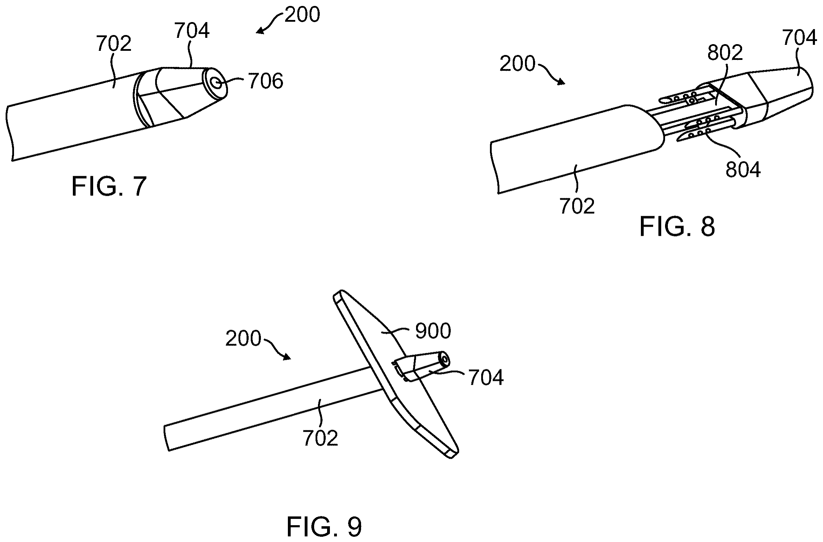

Turning to **FIG. 7**, an isometric view of the distal end of the exemplary vascular device **200** in a low profile condition in accordance with one aspect of the present disclosure is provided. The device **200** may permit placement of the atrial septostomy closure sutures prior to introduction of the cutting implement, that is, a blade septostomy. The entire device **200** may run over the guidewire, which was disposed to the atrial septum through the right atrium.

The vascular device **200** described herein may facilitate creation of a controlled and or adjustable size and position an access incision in a tissue plane such as an atrial septum allowing for a therapeutic instrument or catheter to easily perform a controlled closure of the incision after the procedure is complete. The closure may be tailored to be sealed hemodynamically, or conversely allow for certain amounts of flow. The therapeutic instrument may perform diagnosis and therapeutic intervention to correct atrial fibrillation, perform mitral valve repair, correct septal defects, or perform implantation of a cardiac prosthesis, for example.

The vascular device **200** at a distal end may include a catheter shaft **702** or body, anchor **704,** and guidewire lumen **706.** These components may be made of, for example, a polymeric material. Elastomeric materials may be used to construct the catheter shaft **702** to maximize flexibility. These materials may be used to construct an inner and outer wall of the shaft **702.** Reinforcing structures within the device **200** may be made from metals, such as stainless steel, titanium, or the like. In this embodiment, the reinforcement structure is malleable but retains sufficient force to overcome any forces imparted on it.

The catheter shaft **702,** or delivery catheter, may have a body that is tubular in structure. The shaft **702** may, in one embodiment, have a circular cross-section for housing components. These components may extend towards a proximal end of the vascular device **200.** Left atrial appendage implants described below may be radially collapsible during delivery through the shaft **702.** In one embodiment, the implants may be delivered through 14 French or larger catheters with a radially expandable delivery sheath.

Continuing with **FIG. 7**, the anchor **704** of the vascular device **200** may be delivered to the atrial septum and extendable from the catheter shaft **702.** The anchor **704** and catheter shaft **702** may incorporate components which perform specific functions that enable the delivery of sutures across tissue from a remote location. The anchor **704,** for purposes of this embodiment, may be in a cone-shape. Alternatively, the anchor **704** may have a shape that is, but is not limited to, funneled, rounded, tipped, or peaked. The anchor **704** may have a beveled edge which may conform to the atrial septum.

Traveling or within the catheter shaft **702** and anchor **704** of the vascular device **200** may be the guidewire lumen **706.** The lumen **706** may permit advancement and delivery of the vascular device **200** over the previously placed guidewire. The guidewire, which was described earlier, may enter into the patient through a femoral vein up into the inferior vena cava and into the right atrium. The septal penetrator for placing the initial puncture may be resident within the guidewire, which may be removable therefrom.

**FIG. 8** is an isometric view of the distal end of the exemplary vascular device **200** viewed from a different perspective with a portion of the device **200** advanced revealing components used to puncture tissue and pass sutures in accordance with one aspect of the present disclosure. The anchor **704,** having a cone-shape, may be advanced within the heart of the patient from a tip of the catheter shaft **702.** The amount of distance that the anchor **704** advances may depend on the thickness of the tissue it is crossing and the size of the chamber that it is entering.

The vascular device **200** may be designed to permit or restrict a varying amount of the anchor **704** to travel. The travel may be as small as a few millimeters to upwards of several centimeters. For purposes of this disclosure, it is assumed that the anchor **704** may cross the interatrial septum from the right atrium into the left atrium and the catheter shaft **702** may remain on the right side of the heart within the right atrium. The anchor **704** may be extended such that needles **804** of the anchor **704** are allowed to be hooked or grabbed within the left atrium **304.**

Once the anchor **704** has been advanced, the needles **804** may be exposed. In one embodiment, as shown, four needles **804** may be removably coupled into the anchor **704.** The needles **804** may be rearward facing. For example, the needles **804** may extend towards the catheter shaft **702,** or body, of the vascular device **200** when the anchor **704** has extended into the left atrium. The face of the catheter shaft **702** and anchor **704** may be angled a prescribed amount to permit a more orthogonal contact with the tissue which should promote a more stable interface between the tissue and catheter shaft **702.**

An advancement shaft **802** for the anchor **704** of the vascular device **200** may be rectangular in shape and travel through a corresponding rectangular-shaped lumen within the catheter shaft **702** in order to maintain a precise rotational alignment with the catheter shaft **702.** Typically, the alignment between the anchor **704** and catheter shaft **702** is maintained as it permits device functionality. The advancement shaft **802** may be extended and retracted at a proximal end of the vascular device **200,** which as shown above may be located at the percutaneous puncture or incision point.

Referring to **FIG. 9**, an isometric view of the distal end of the exemplary vascular device **200** with the portion of the device **200** advanced through the tissue **900** of the atrial septum in accordance with one aspect of the present disclosure is provided. The vascular device **200** has been placed through a representative section of tissue **900.** The anchor **704,** having a cone-shape, has been advanced across the puncture site of the tissue **900** which was performed earlier by the septal penetrator. The catheter shaft **702** may be larger than the puncture created by the septal penetrator, thus precluding the catheter shaft **702** from entering into the tissue **900.** This may allow for the tissue to be tight or secured around the narrow aspect of the apparatus.

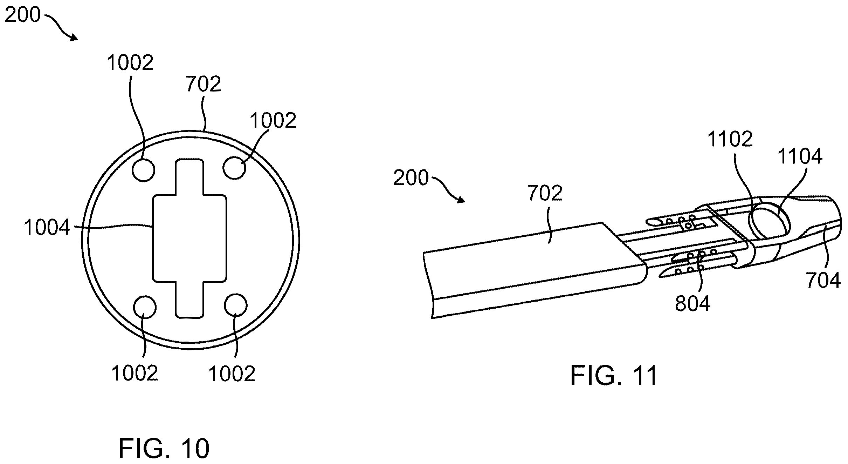

**FIG. 10** is a cross sectional view of the exemplary vascular device **200** having an illustrative lumen configuration in accordance with one aspect of the present disclosure. The catheter shaft **702** may include several channels or lumens that permit certain components to pass therethrough. As an example, four lumens **1002** having equal diameters may permit snares to grab, hook or loop the needles removably coupled to the anchor with tethered sutures to pass through them. The central rectangular-shaped channel **1004** may permit the rectangular-shaped advancement shaft of the anchor to travel through it. The configuration may maintain an alignment of the four needles and cutting implements, which will be shown later, within the catheter shaft **702.**

Different configuration to the cross section of the vascular device **200** may be implemented depending on suture placements. For example, more than four lumens **1002** may be channeled through the catheter shaft **702** with each being equidistant from the center. Advantageously, the lumens **1002** may provide a proper spacing to the initial puncture such that sutures management may be had. Punctures used to capture the needles into the lumens **1002** may be placed such that no unnecessary tearing of tissue is made yet still proper suture placement is performed. The shown configuration may allow for two sutures through four needles, but other configurations may exist and are within the scope of the present disclosure. In one embodiment, apertures may radially surround the central lumen where the user may selectively advance any number of needles/sutures through. The sutures may be loaded from the proximal end in any configuration the user has chosen.

**FIG. 11** is an isometric, sectioned view of the distal end of the exemplary vascular device **200** revealing the inner geometry of components therein in accordance with one aspect of the present disclosure. For illustrative purposes, a section of the anchor **704** has been removed permitting a view of a bundled suture **1102** that is stowed in a recess channel **1104** of the cone-shaped anchor **704.**

Ends of the suture bundles **1102** may be coupled to two needles **804** on opposite ends. Two suture bundles **1102,** as shown, may have four needles **804.** The suture bundles **1104** may be placed on opposite sides of one another delineated, or separated, by the advancement shaft. The needles **804** on both sides may be engaged simultaneously with the recess channel **1104** unspooling both suture bundles **1102.** The recess channel **1104** may rotate for two different bundled sutures **1102** while the needles **804** are being drawn into the catheter shaft **702.**

The recess channel **1104** may be shaped to permit the unspooling and release of the suture bundles **1102.** In one example, sutures of the suture bundles **1102** may be spooled into the recess channel **1104** which may be held taught within the anchor **704.** When the needles tethered to the suture bundles **1102** are pulled, the suture bundles **1102** may be unspooled. The sutures of the suture bundles **1102** may be released from the anchor **704** after the suture bundles **1102** are pulled a predetermined amount by the needles **804** which are drawn into the catheter shaft **702.** This amount may be, for example, a couple of centimeters.

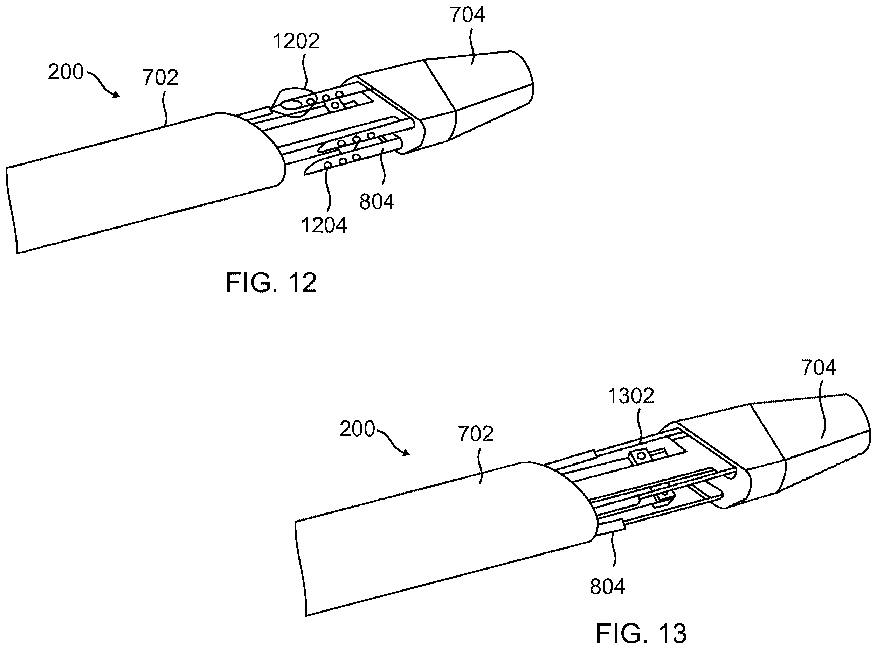

With reference to **FIG. 12**, an isometric view of the distal end of the exemplary vascular device **200** revealing snares **1202** that have been advanced out of a body of the vascular device **200** in accordance with one aspect of the present disclosure is provided. Four snares **1202** may be used to grab, hook or loop the suture bundles with each suture bundle having on opposite ends needles **804** for snaring. Small punctures may be made for the snares **1202** to pass through the tissue, or the snares **1202** themselves may have a tip capable of puncturing the tissue. The snares **1202** may be placed through the catheter shaft **702** at a proximal end and out of the previously described lumens. Once the needles **804** have been captured, the snares **1202** may be retracted through the catheter shaft **702.**

In one example, recesses **1204** placed within the needles **804** may be used by the snares **1202.** These recesses **1204** may be slanted and directed towards the anchor **704** of the vascular device **200.** When the snares **1202** are pushed through the catheter shaft **702,** a hook of the snare **1202** may be pulled and tethered against the recess **1204.**

A mechanism may be used at a proximal end of the catheter shaft **702** to push multiple snares **1202** therethrough simultaneously. The snares **1202** may grab and then pull the needles **804** through the tissue at the same time. In an alternative embodiment, the snares **1202** may be individually pushed into the shaft **702** to capture or hook a single needle **804** at one time through its recess **1204.** In one embodiment, two snares **1202** may work in tandem to pull two corresponding needles **804** through the shaft **702.** The two needles **804** may be connected to opposite sides of the suture bundle. The needles **804** may be trimmed from the suture after being pulled into the shaft **702.**

Snares **1202** may be made of a variety of materials. For example, the snares **1202** may be made of a radiopaque platinum coil and tip for enhanced visibility. The snare **1202** may include a helical loop design for a smaller profile yet a longer reach than right-angle loops. A durable cobalt chromium loop may add strength and retain its shape. The snare **1202** may have a loop that varies in size: 1 mm, 2 mm and 3 mm loop diameters for clinical versatility.

**FIG. 13** is an isometric view of the distal end of the exemplary vascular device **200** after four suture needles **804** and suture ends have been passed through the tissue **900,** snared and pulled through the length of the device **200** in accordance with one aspect of the present disclosure. The catheter shaft **702** of the vascular device **200** may receive the needles **804** after being pulled by the snares. From the bundled sutures with the pull of the needles **804** coupled on opposite ends, sutures **1302** may be unspooled from the recess channel and tensioned.

The suture **1302,** in one embodiment, may be made of finely woven nylon material. Other materials may be used such as, but not limited to, polypropylene, silk or polyester. The sutures **1302** may be made of a sturdy, but bendable material. The sutures **1302** may be in a "U" or "C" shape. The sutures **1302** may be soaked in a sterile mineral oil immediately prior to its use. The edges of the suture **1302** may be sutured easily to margins of an incision in the atrial septum. The suture may be made of a high temperature resistant material to prevent damage if it is in contact with the cutting implement.

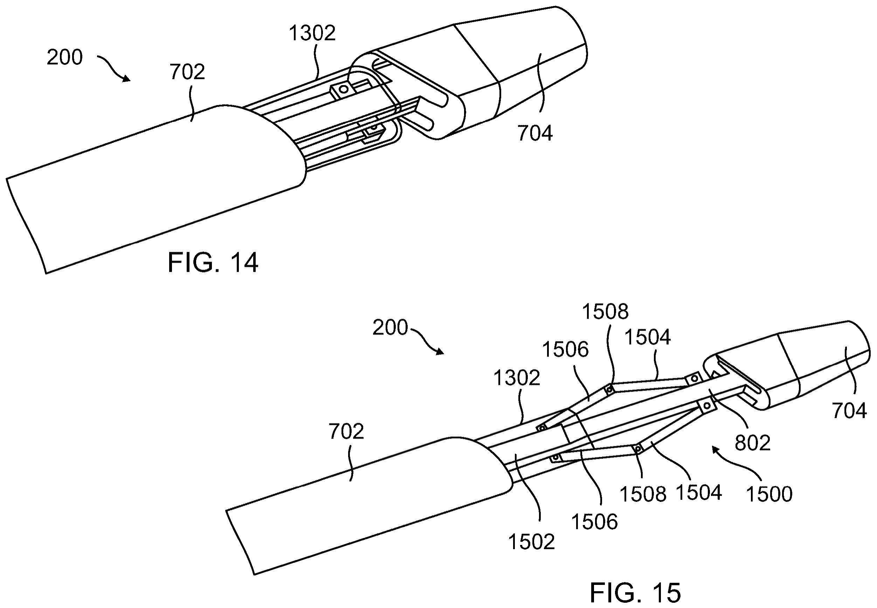

**FIG. 14** is an isometric view of the distal end of the exemplary vascular device **200** after the suture **1302** has been pulled completely through the length of the device **200** and is now pulled taught against the tissue in accordance with one aspect of the present disclosure. The sutures **1302** may be fully unspooled from the recess of the anchor **704.** The ends of the suture **1302** may be available to the user through the proximal end of the catheter shaft **702** of the vascular device **200.** In operation, the suture lines may be slackened or tightened according to a user's need at a particular time.

With reference now to **FIG. 15****,** an isometric view of the distal end of the vascular device **200** after a cutting implement **1500** has been partially advanced revealing the cutting elements **1506** in accordance with one aspect of the present disclosure is provided. Once the sutures **1302** are pulled against the tissue, such that they may not be cut, the anchor **704,** having the cone-shape, may be further advanced into the left atrium through the advancement shaft **802.** The shaft **802** may be extend through the catheter shaft **702** of the vascular device **200** towards and through the puncture in the atrial septum. The advancement shaft **802** may be extended through the proximal end of the vascular device **200.**

By advancing the shaft **802** through the tissue of the atrial septum, a second, rectangular-shaped telescoping cutting implement **1500** may be sent through the rectangular-shaped channel of the catheter shaft **702.** The cutting implement **1500** may include an expansion actuating shaft **1502** that may telescope over the advancement shaft **802** for the anchor **704.** That is, the expansion actuating shaft **1502** may slide over the advancement shaft **802** of the anchor **704.**

The expansion actuating shaft **1502** may be advanced through the puncture and be located within the left atrium extending the cutting implement **1500.** When the expansion actuating shaft **1502** is pushed forward, the cutting implement **1500** with a linkage system **1504** is exposed. A distal end of the cutting implement **1500** may be temporarily locked to a top portion of the advancement shaft **802** of the anchor **704** while a proximal end of the cutting implement **1500** may be connected to a lower portion of the expansion actuating shaft **1502.**

The linkage system **1504** may bow outward and expand the cutting elements **1506** to a length much greater than the diameter of the vascular device **200** when the anchor **704** is retracted and the expansion actuating shaft **1502** is held in place. The linkage system **1504** may bend at symmetrical points **1508** when the advancement shaft **802** is retracted. The cutting elements **1506,** as shown, are positioned behind the anchor **704** facing towards the catheter shaft **702.** In operation, the linkage system **1504** may remove any potential to cut the sutures **1302,** which are parallel thereto, as the cutting elements **1506** are expanded.

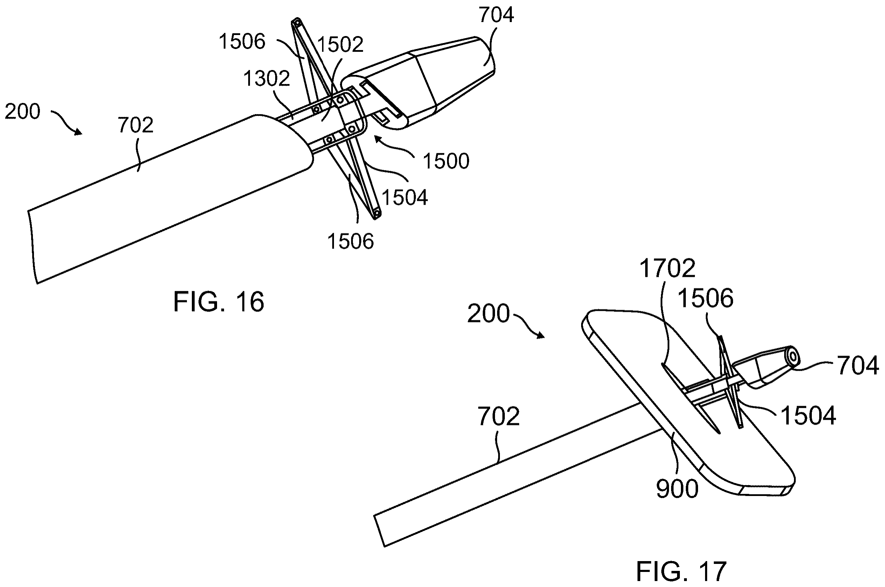

**FIG. 16** is an isometric view of the distal end of the vascular device **200** with the cutting implement **1500** expanded in accordance with one aspect of the present disclosure. The anchor **704,** in one configuration, has been retracted covering the advancement shaft. When this is performed, the top of the cutting implement **1500** is held and lowered with the anchor **704** and the expansion actuating shaft **1502** holds a bottom portion of the cutting implement **1500** in place. This may cause a bend in the cutting implement **1500** within the linkage system **1504** and expose the cutting elements **1506.** The cutting elements **1506** may be perpendicular to the shafts, such that the cutting elements **1506** expand past a diameter of the catheter shaft **702.**

The parallel alignment of the cutting elements **1506** relative to the sutures **1302** may be made so that the cutting elements **1506** do not inadvertently cut the sutures **1302.** Multiple cuts or incisions may be made by cutting elements **1506** by slicing through the tissue and pulling through the cutting elements **1506** back to the left atrium. The processes may be repeated depending on the number of incisions needed. Accordingly, the number and location of sutures/needles may vary depending on the size and number of incisions made.

When completed, the cutting elements **1506** may be retracted by advancing the anchor **704.** The linkage system **1504** may be collapsed through this advancement and the system **1504** may condense into a narrow channel without the cutting elements **1506** exposed. The lock coupling the top of the advancement shaft and the cutting implement **1500** may be removed. The cutting implement **1500** may then be pulled through the catheter shaft **702** without moving the anchor **704.** After removing the cutting implement **1500,** the vascular device may be removed leaving the sutures **1302** in place.

Other technique or devices may be used to expand and collapse the cutting elements **1506** such that no tissue is inadvertently cut when the anchor **704** of the vascular device **200** is being used in the left atrium. The linkage system **1504,** allowing the cutting elements **1506** to be used, may come in a variety of forms and is not necessarily limited to that shown in this embodiment. For example, the linkage system **1504** may entirely reside on the expansion actuating shaft **1502** whereby mechanisms on the proximal end may be used to expand and collapse the cutting elements **1506** without the need to retract the anchor **704.** This may use a separate knob, pull-wire, or the like to expand and collapse the cutting elements **1506.** Other variations may exist and are within the scope of the present disclosure.

With reference now to **FIG. 17****,** an isometric view of the exemplary vascular device **200** positioned to cut the tissue **900** in accordance with one aspect of the present disclosure is provided. The vascular device **200** may be angled at a prescribed amount to permit a more orthogonal contact with the tissue **900.** This may promote a more stable interface between the tissue **900** and catheter shaft **702.**

The distal end of the catheter shaft **702** may be angled to conform to the tissue **900.** The shaft **702** typically does not go through the initial puncture or the slit **1702** created by the cutting elements **1506.** The anchor **704** of the vascular device **200,** however, may extend through the puncture and into the left atrium. The linkage system **1504** may be expanded and the cutting elements **1506,** which may be in the form blades, may be used to cut a slit **1702** through the tissue **900.** The length of the slit **1702** may be controlled by how much the linkage system **1504** is expanded. The vascular device **200** may be rotated to produce other slits **1702** or combinations of slits **1702.**

In operation, the anchor **704** of the vascular device **200** may advance through the initial puncture. Needles may extend towards the catheter shaft **702.** The sutures may then be brought towards the tissue **900** from a backend before any incision **1702** is made. The sutures may then be managed after the incision **1702** and the therapeutic instrument has been used.

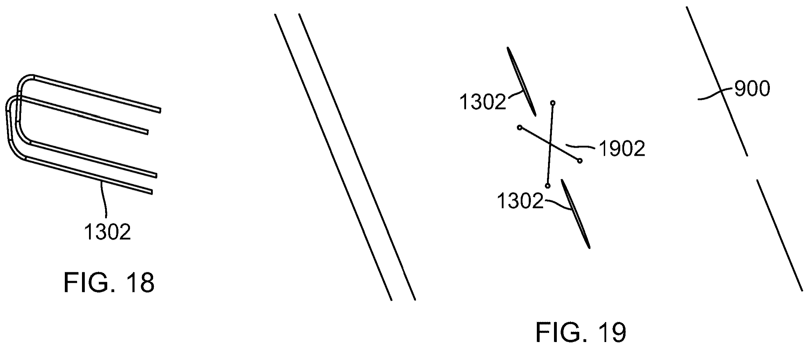

**FIG. 18** is an isometric view of sutures **1302** that would be inserted into the tissue **900** in accordance with one aspect of the present disclosure. The two sutures **1302** may correspond to those that were unspooled from the recess of the anchor. After removing the vascular device, two lengths of sutures **1302** may remain in place behind the atrial wall that extends from the left atrium.

In addition, the guidewire previously used by the vascular device may also be left in place. The guidewire may be used by a larger bore device, such as a therapeutic instrument, that may now travel over the guidewire and gain easy access into the left atrium through the slit and run adjacent to the previously placed sutures. Once the large bore device has been removed, the free ends of the suture **1302** may be knotted and pushed towards the tissue to create a closing force. This may reduce the size of the cut or hole within the tissue, which the large bore device used. Advantageously, this may prevent or minimize the amount of hemodynamic communication between chambers and allow the user to leave behind a simple knot on the interatrial septum. Typically, the hole may close in the short term. In the long term, this may be used to permit a subsequent access into the left atrium if another catheter-based procedure or intervention is needed for the patient.

**FIG. 19** is an isometric view of an incision after the tissue **900** is engaged with the suture **1302** in accordance with one aspect of the present disclosure. A close-up provides a view of the tissue **900** after a knot **1902** has been tied. A slit, which was created by the cutting implement has been cinched down in the middle by the knot **1902,** which may reduce or eliminate an amount of hemodynamic flow and communication between chambers. For illustrative purposes the knot **1902** may be represented by a simple "X" configuration. Alternatively, the knot **1902** may be one of several different types of surgical knots that may be tied and pushed down the vascular device via a remote location.

In one embodiment, the knot **1902** may be formed and advanced with a knot pusher having a pusher rod fitted with a distal side port and severing member in the form of a sharpened outer sheath. The knot **1902** may hold an associated patch in place where an excess line may be trimmed by the shearing action of the pusher rod distal side port and the distal sharpened portion of the severing member. The excess line and other elements may be removed from the catheter.

Knot pushers that are known in the art include Edwards ThruPort knot pusher; Medline Endoscopic Pushers; Laparoscopic Knot Pushers by Cooper Surgical.

Arthrex offers several options: The Single-Hole Knot Pusher provides a simple method to advance sliding knots and half-hitches. This closed end knot pusher has a modified handle that provides an ergonomic feel. The distal tip has also been modified for easier advancement of slipknots and half-hitches. The 6th finger was designed to tie the surgeon's knots and allows the surgeon to apply and maintain tension to the first throw while advancing subsequent throws. The CrabClaw incorporates an opening jaw to allow intraarticular capture of suture.

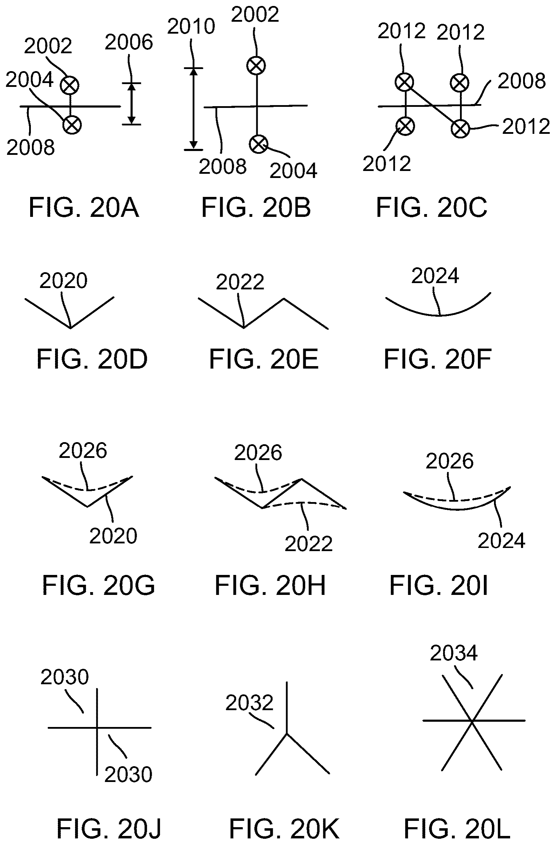

A simple incision with closure was described beforehand. Turning to **FIGS. 20A-L,**various illustrative cut patterns that may be created using the vascular device in the atrial septum which may be made from at least one cutting implement that may be rotated and used a single or multiple times in accordance with one aspect of the present disclosure are provided. The embodiments may create the various cutting shapes in the atrial septum. These shapes may be created with multiple cutting arms, or using one cutting arm that is rotated and used multiple times, such as the cutting implement shown above. The length of the incision may be controlled by the user through activation of the adjustable cutting implement. The incision may take a number of different geometries commonly used to facilitate both passage of the therapeutic device and closure of the tissue plane post therapy.

With reference to **FIGS. 20A-20C****,** apposition of the tissue edges may be controlled by the nature of the location of the anchors or sutures **2002** and **2004.** To increase overlap or tissue apposition, a first distance **2006** between the sutures **2002** and **2004** may be increased to a second distance **2010.** Another method or technique may be to add additional suture locations or sutures **2012** at various and intersecting paths. The incision **2008** may be in the middle of the sutures **2002, 2004** and **2012.**

The incision **2008** may take the form of many patterns such as a straight cut, v cut **2020,** zig zag **2022,** or crescent arc **2024,** to name a few, and as shown in **FIGS. 20D-F.**These cuts may then be coupled together with other shapes yielding multiple flaps of tissue **2030, 2032,** or **2034** shown in **FIGS. 20J-L.** The various configurations may be advantageous depending on the shape and size of the device needed to pass through and or the type or amount of closure that is desired post procedure. Some incision shapes such as **FIGS. 20D-F**may facilitate a tissue plane overlap **2026** upon closure due to the shape of the cut and the tension of the tissue before and during healing. This overlap may greatly aid the healing of the tissue edges together. That is, in **FIGS. 20G-I** what is shown is the tissue plane overlap **2026** from the different incision shapes **2020, 2022,** and **2024.**

The embodiments described herein manage the variables to control the closure of the incision **2008.** The tissue edges may be managed by having control members in place before the incision **2008** is made. For example, and as shown above, having sutures **2002, 2004** and **2012** in place before incisions **2008** are made by the cutting implement may secure the tissue. In one embodiment, control features may be applied after the incision **2008** is made.

The tissue edge position and apposition of those edges relative to each other may be controlled to manage an amount of tissue overlap, apposition pressure, and amount of residual flow after a closer is applied. This control may be done by controlling where the suture lines are positioned relative to the incision. An example of this, is the distance of the suture lines farther away from the incision edge may cause more tissue bunching and/or tissue overlap. Increasing the number of tissue anchors and/or suture passing locations may increase the amount of tissue apposition along the length of the incision. The amount of tension or pressure applied to the suture tension lines may further affect the amount of closure on the incision **2008.** These mechanisms may be managed in real-time and monitored under echo flow monitoring and/or fluoroscopy visualization.

In one embodiment, a mechanism may be used that would leave no long-term implant left in the patient. The mechanism may be designed to either seal the tissue, have the tissue heal in a sealed state, or have the tissue heal in a partially sealed state. The percentage of the hemostasis may be adjusted by varying the application of the mechanism. The mechanism may be designed to secure the tissue through various time points. These time points may correlate to various tissue healing cascade points such as time for tissue to coagulate, adhere, endothelialize, and scaring.

An absorbable body may be left that would facilitate the closure and be absorbed by the body over time. In one embodiment, the absorbable body may be removed from the body at a later point in time. The closure may be fully or partially engaged into the tissue plane near the incision **2008** before the incision **2008** is made. In another embodiment, applying the closure fully or partially engaged into the tissue plane may be near the incision **2008** after the incision is made. In one embodiment, the closure mechanism may be applied fully or partially engaged into the tissue plane near the incision **2008** after the incision **2008** and therapeutic instrument is removed from the incision **2008.**

The cutting implements described herein may be used to control the condition of the tissue edges based on the method of creating the incision in the tissue. The methods of creating the incision may include, but are not limited to, a sharp blade made from durable material such as metal or ceramic, electrocautery techniques, RF energy, plasmajet vaporization, ultra-sonics, high voltage vaporization, controlled dilation, heat, cold, and others. A state of the cells on the edge of the cut surface may be controlled to optimize the desired healing cascade utilizing these various methods of incision creation.

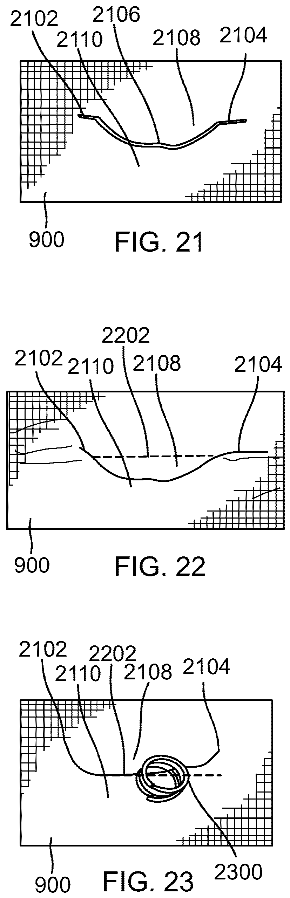

**FIG. 21** is an isometric view of an illustrative cut pattern **2106** for incision to promote tissue edge apposition in accordance with one aspect of the present disclosure. A cut pattern **2106** may be defined by a first end **2102** and second end **2104.** A cut pattern **2106** may be made into the tissue **900.** The inside edge **2108** is separated from the outside edge **2110.** This may be a cut pattern **2106** of a straight and arc combination for incision to promote tissue edge overlap and apposition.

**FIG. 22** is an isometric view of the illustrative cut pattern for incision to promote tissue edge apposition, while under slight tension, demonstrating tissue edge control and overlap of edges with tension applied in accordance with one aspect of the present disclosure. This demonstrates overlap of the inside edge **2108** and outside edge **2110** with yield overlap **2202** in the tissue **900** caused by the cut pattern between the first end **2102** and second end **2104.**

**FIG. 23** is an isometric view of the cut patterns for incision to promote tissue edge apposition and a helical anchor **2300** controlling tissue edges in accordance with one aspect of the present disclosure. Tension may be applied in the direction of the ends **2102** and **2104** of the cut. The inside edge **2108** and outside edge **2110** with yield overlap **2202** in the tissue **900** may be secured with a helical anchor **2300,** or the like.

Previously, a first embodiment of a vascular device was described. **FIGS. 24-38** describe a second embodiment of a vascular device **2400** with additional illustrative incisions. It will be understood from the present disclosure that components with these embodiments may be interchanged, added or deleted based on a reasonable configuration. New embodiments using these modifications are within the scope of this disclosure.

Turning to **FIG. 24****,** an isometric view of an illustrative expandable RF cutting implement **2410** with four expandable members **2416** in accordance with one aspect of the present disclosure is provided. The cutting implement **2410** may be between a catheter shaft and a distal anchor, as previously described. While four cutting members **2414** are shown, fewer or more may be used with each cutting member **2412** equidistant from one another.

The cutting implement **2410** may be expandable and collapsible through similar linkage systems described above. The cutting implement **2410** may be collapsed when advanced through the initial puncture and expanded after passing through the tissue. The cutting implement **2410** may have four expandable members **2414** connected to four cutting members **2412.** The cutting members **2412** may be provided on a proximal end of the cutting implement **2410** such that the cutting implement is pulled back towards the tissue to make cuts.

The cutting members **2412** may extend radially from the center of the cutting implement **2410.** The width of the cutting members may vary in width to change the incision length based on a French size of the delivery catheter. The cutting implement **2410** may also include a tip piercing device **2416** at a distal end of the cutting implement **2410.** This may be used to puncture the tissue. The cutting members **2412** and tip piercing device **2416** may use mechanical or electrical energy. The mechanical or electrical energy may come from at least one of a blade, ceramic, electrocautery technique, RF, plasmajet vaporization, ultra-sonic, high voltage vaporization, controlled dilation, heat and cold. In one example, the cutting members **2412** and tip piercing device **2416** may both use mechanical energy. Alternatively, they may use both electrical energy. In yet another variation, the cutting members **2412** and tip piercing device **2416** may use different types of energy. The cutting implement **2410** along with its members **2412** and arms may radially expand in a controlled manner or plane such that they minimize or prevent the likelihood that the cutting implement **2410** inadvertently cuts or negatively impacts the previously placed closing sutures.

**FIG. 25** is an isometric view of the exemplary vascular device **2400** with the expandable cutting implement **2410** having a tip for piercing the tissue **900** in accordance with one aspect of the present disclosure. A tip piercing device **2416** may be coupled to the cutting implement **2410** and may be pushed through the tissue **900** to create a puncture within the tissue **900.** The puncture may be made through a mechanism on a proximal end allowing a physician to control the vascular device **2400.** The cutting implement **2410** may be in a retracted state before advancing through the tissue **900.**

A catheter shaft **2504,** or delivery catheter, may house, but is not limited to, the cutting implement **2410** and anchor mechanisms **2502.** The anchor mechanisms **2502** may be equidistant from the center of the catheter shaft **2504.** While four anchor mechanisms **2502** are shown, fewer or more exist depending on a closure strategy of incisions made into the tissue **900.**

The vascular device **2400,** while not shown, may include visualization tools for determining a location of the device **2400** within the patient. In one embodiment, a sensor may be affixed to the device **2400** to determine a location and orientation of the catheter. Alternatively and/or additionally, an independent tracking system may be based on ultrasound, impedance or fluoroscopy tracking. In the case of impedance, electrical potential generated by electric field generators may be detected by the existing electrodes. In the case of fluoroscopy, electrode location may be detected by an image processing scheme that identifies and tracks the electrodes and/or opaque markers located on the device **2400.**

**FIG. 26** is an isometric view of the exemplary vascular device **2400** with the illustrative expandable cutting implement **2410** placed beyond the tissue **900** in accordance with one aspect of the present disclosure. The cutting implement **2410,** in its retracted state, may be pushed through after the puncture is made to the tissue **900.** The cutting implement **2410** may be sent through by an advancement shaft which may be controlled at a proximal end whereas the catheter shaft **2504** is not distributed therethrough.

After the cutting implement **2410** has been distributed into the left atrium, the cutting implement **2410** may be expanded. The expandable members **2414** may be radially extended from its center which spreads to a larger diameter than a diameter of the device **2400** itself.

An anchor mechanism **2502** within the catheter shaft **2504** may be used to push delivery mechanisms, which will be described below. The anchor mechanisms **2502** may be distributed within the catheter shaft **2504** and enclosed within lumens. The anchor mechanisms **2502** may surround the centralized cutting implement **2410** and be equidistant from one another.

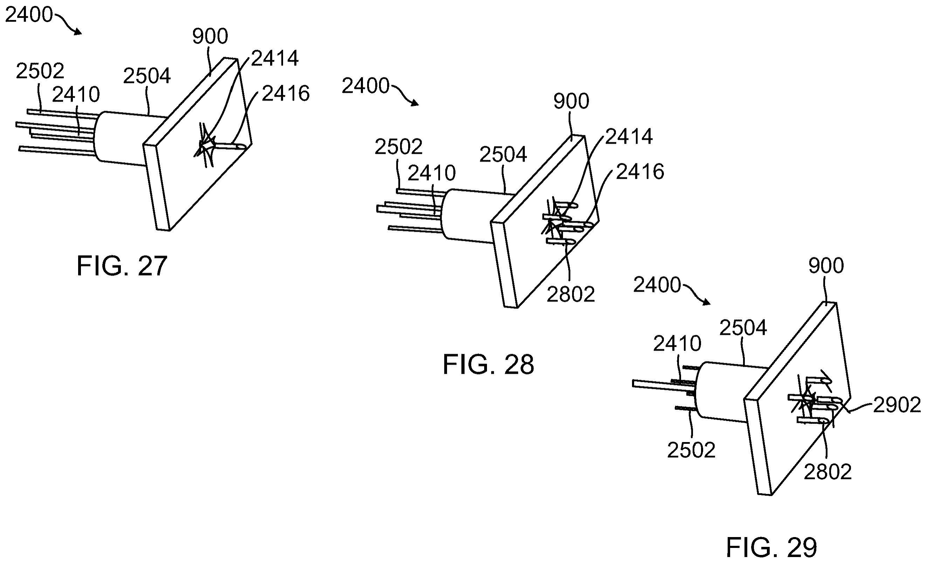

Referring to **FIG. 27****,** an isometric view of the exemplary vascular device **2400** with the illustrative expandable cutting implement **2410** making incisions into the tissue **900** in accordance with one aspect of the present disclosure is provided. After the expandable members **2414** are extended, the advancement shaft may be pulled back, along with the tip piercing device **2416,** toward the catheter shaft **2504** to make incisions in the tissue **900.** These incisions may be made in a backplane of the left atrium. The cutting implement **2410** may be extended, rotated, and then retracted to make additional incisions into the tissue **900.** During this time, the anchor mechanisms **2502** may be held stationary.

**FIG. 28** is an isometric view of the exemplary vascular device **2400** with the illustrative expandable cutting implement **2410** advancing anchor delivery mechanisms **2502** in accordance with one aspect of the present disclosure. When the anchor mechanisms **2502** are pushed through the catheter shaft **2504,** it may extend through the tissue **900** to the other side, that is, the left atrium. The anchor mechanisms **2502** may be coupled to a delivery mechanism **2802** that may be transferred through the tissue **900.** The delivery mechanisms **2802** may have tissue piercing points.

Four delivery mechanisms **2802,** coupled to four anchor mechanisms **2502,** may pierce the tissue **900.** Fewer or more combined structures may be present within the vascular device **2400.** The delivery mechanisms **2802** may use similar energies to the expandable members **2414** and tip piercing device **2416.** That is, combinations of mechanical and/or electrical energies may be used.

**FIG. 29** is an isometric view of the exemplary vascular device **2400** with the illustrative expandable cutting implement **2410** with push anchors **2902** further inserted to advance the anchor mechanisms **2502** in accordance with one aspect of the present disclosure. The anchor mechanisms **2502** may advance the delivery mechanisms **2802** within the catheter shaft **2504** into and beyond the tissue **900,** that is, within the left atrium. The push anchors **2902** may thereafter be deployed by the delivery mechanisms **2802.** The push anchors **2902** may be used to secure the tissue **900,** and its surrounding area. The anchors **2902** may be made from PLGA, PLLA, nylon, polyester, PEEK, or other biocompatible material. It should be noted that cutting implement **2410** may be advanced into the tissue **900** after the push anchors **2902** are set in place.

Other anchors may be used for securing the tissue **900.** For example, tissue anchor lines may be utilized. These may include, but are not limited to sutures, toggles, helical structures, grabbing devices, inverting clips, expanding structures, mesh structures, stent-like structures, patch structures, clips, expandable valves, and suture knot configurations.

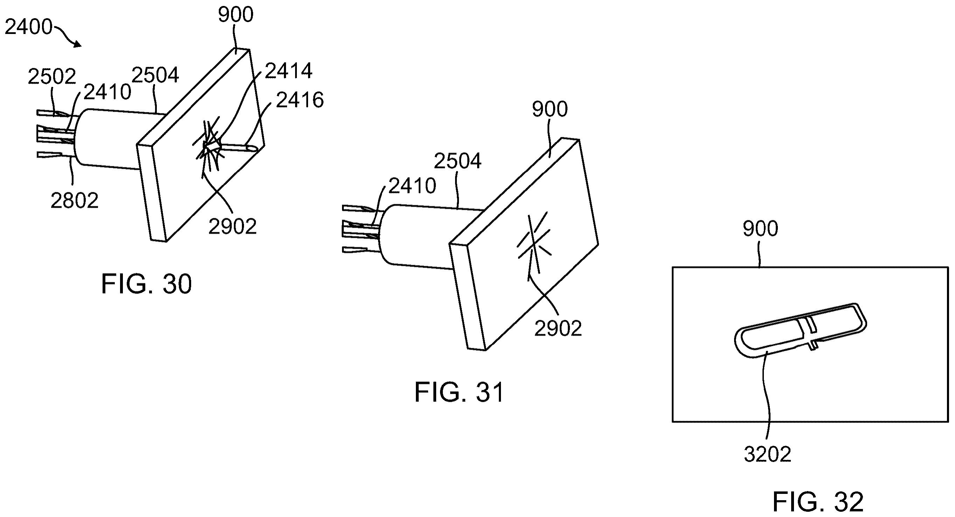

**FIG. 30** is an isometric view of the exemplary vascular device **2400** having the delivery mechanisms **2802** removed in accordance with one aspect of the present disclosure. The anchor mechanism **2502** coupled to the delivery mechanisms **2802** may be pulled at a proximal end of the catheter shaft **2504.** This may retract the delivery mechanism **2802** from the left atrium **302** into the right atrium **106.** The cutting implement **2410** with its expandable members **2414** and tip piercing device **2416** may still be inserted into the left atrium of the patient. The anchors **2902** may be left against the tissue **900.** These may be embedded therein.

**FIG. 31** is an isometric view of the exemplary cutting implement **2410** removed from the tissue **900** leaving tissue anchors **2902** against it in accordance with one aspect of the present disclosure. The cutting implement **2410** may be removed through the catheter shaft **2504** of the vascular device **2400.** The catheter shaft **2504** may still be placed in the right atrium of the patient at this time.

Referring to **FIG. 32****,** an isometric view of an exemplary toggle **3202** within the tissue **900** in accordance with one aspect of the present disclosure is provided. The toggle **3202** may be pushed or advanced through the tissue longitudinally from the right atrium into the left atrium. The toggle **3202** may be shifted horizontally and secured on the septal wall to hold the tissue in place. The toggle **3202** may be made of similar materials to the anchor, which may be biodegradable. The presence of this closure apparatus or of others herein disclosed are of a size and location that they will not preclude another access procedure in the future.

Multiple cutting implements were described beforehand. These implements, as well as those described below, may use mechanical or RF energy. When using electrical energy, an amount of exposed metal may be minimized through insulation such that the exposed metal may only exist in the desired tissue cutting region of the tool. The smaller the amount of exposed metal, a better cutting effect on the tissue may be realized. Advantageously, this may use less power. To achieve the best cutting effect, the operator may ensure the cutting region is in good mechanical contact with the target tissue.

The initial septal puncture site and the septal cut may be performed using separate applications of energy. For example, using electrical energy, a first puncture may be performed using a first circuit and a larger cut may be achieved using a second circuit. If the first puncture is done separately from the cut, the operator may then rotate the cutting implement to align a cutting arm with a direction they intend to cut. If performing the second cut after the initial puncture by advancing the cutting tool from the right atrium to the left atrium, it may be beneficial to have the cutting regions of the initial puncture and the second larger cut overlap so there is no chance of a piece of tissue not being cut.

If the tool has symmetric cutting arms on either side of the center puncture element, the center of the overall cut may be at the intended puncture site, and not shifted in one direction. This is important for the success of a subsequent procedural step that may require being a certain distance above the target structures.

The following cutting implements may create a continuous cut form a center puncture site to the edge of the cut. The intent of these implements is to cut all the way from the center to the edge. The adjustability or expandability of the cutting implement may be achieved using a pull-wire/ring mechanism, such that the cutting implement may be compressed and bowed outward as the wire is pulled, which was described above. It may also be performed using a spring, or by using a shaped tool made of shape memory alloy.

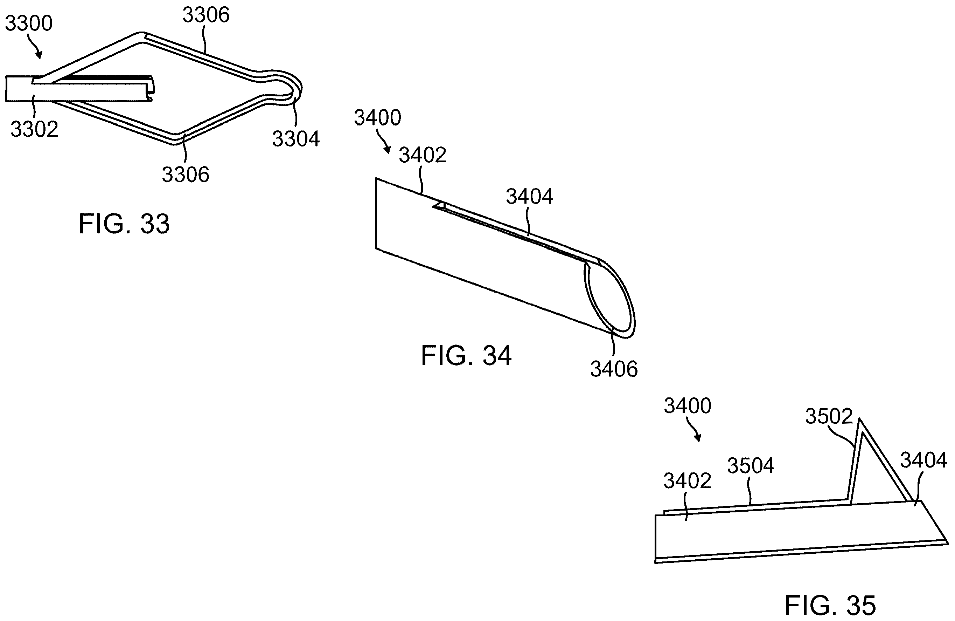

Turning now to **FIG. 33****,** an isometric view of an illustrative cutting implement **3300** having an atraumatic tip **3304** deployed from an exemplary vascular device in accordance with one aspect of the present disclosure is provided. The cutting implement **3300** may be deployed from a distal end of a tubular member **3302** with electrical insulation selectively removed. This cutting implement **3300** may be fixed in size or adjustable through mechanism design.

The design may incorporate an atraumatic tip **3304** that may be used for finding the fossa ovalis or other desired target location. It also has cutting surfaces **3306** that may extend to both sides of the atraumatic tip **3304,** symmetrically. In these types of embodiments, the initial septal puncture and slit creation may be performed in one motion with the same continuous cutting surface **3306,** that is, they may be part of the same circuit for delivering energy, and they may also be on individual circuits. If the puncture/cutting energy is to be RF, microwave, or other electrical energy, insulation may be strategically removed from the metal structure. The amount of insulation removal, or alternatively metal exposure, may be varied to optimize performance. It may wrap entirely around the cutting arm, for example, or may be present in a narrow line along the length of the cutting surface arm. The goal may be to ensure the cutting surface being energize and in good contact with the tissue, with minimal direct communication to a blood pool.

**FIG. 34** is an isometric view of an illustrative cutting implement **3400** having a slit **3404** in a sheath **3402** deployed from an exemplary vascular device in accordance with one aspect of the present disclosure. A cutting tool that incorporates one cutting arm may expand radially through the slit **3404** in the needle type sheath **3402,** which will be shown below. In this embodiment, with only one cutting arm, the size of the arm may be fixed or adjustable through a mechanism, for example, using a pull-wire, spring, or the like. A distal portion **3406** of the cutting implement **3400** may be used for the initial puncture. Unlike the embodiments described above, the initial septal puncture site and slit creation are a part of separate parallel circuits, if electrical energy is used for cutting the tissue.

**FIG. 35** is a side view of the illustrative cutting implement **3400** having a cutting element **3502** extending from the slit **3404** in the sheath **3402** in accordance with one aspect of the present disclosure. The expandable cutting arm **3504** is a part of a pull-wire that may run the length of the catheter and the puncture needle face is a part of another.

In one embodiment, the initial puncture needle may be entirely insulated, with only the distal region having exposed metal for energy delivery to the tissue. The expandable cutting element **3502,** in the form of a radially extending arm, may have exposed metal circumferentially, or be mostly insulated with a thin line of exposed metal running along the cutting surface (or any number of patterns for exposing minimal surface area of the metal). It may be possible and more desirable for these two different cutting surfaces to be energized at the same time with one switch or through different switches on the handle end to allow for energy application at different times during the procedure. The benefit of only puncturing with the needle first is that it may create an anchor point in the tissue. Once this initial puncture is made, the operator may rotate the cutting implement **3400** until the expandable cutting element **3502** aligns with where they want the length of the cut to go.

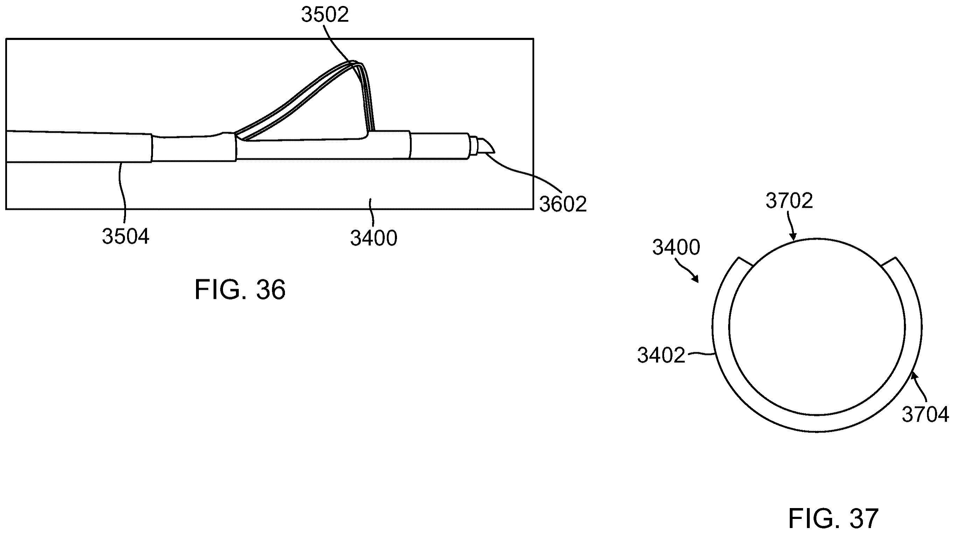

**FIG. 36** is an isometric view of a distal end of the exemplary cutting implement **3400** in accordance with one aspect of the present disclosure. The expandable cutting arm **3504** for the cutting element **3502** may be a wire that runs the length of the catheter. A puncture needle **3602** may be part of another mechanism to be actuated. The cutting element **3502** may have exposed metal circumferentially, or be mostly insulated with a thin line of exposed metal running along the cutting surface. In operation, the expandable cutting arm **3504** may be pushed up and down to change the shape of the cutting element **3502.**

The initial puncture needle **3602** may be entirely insulated, with only the distal region having exposed metal for energy delivery to the tissue. In one embodiment, the cutting implement **3400** may have two different cutting surfaces to be energized at the same time with one switch or through different switches on the handle end to allow for energy application at different times during the procedure. The benefit of puncturing with the needle **3602** first is that it may create an anchor point in the tissue. Once the initial puncture is made, the operator may rotate the tool until the expandable cutting arm aligns with where they want the length of the cut to go.

Furthermore, the cutting element **3502** may be retracted inward when the expandable cutting arm **3504** is pulled in a proximal direction. The cutting element **3502** may be retracted towards the center of the cutting implement **3400.** The energy applied to the cutting element **3502** may be removed to prevent inadvertent cutting.

**FIG. 37** is a cross-sectional illustration of the cutting implement **3400** in accordance with one aspect of the present disclosure. A schematic showing how the cutting arms of the embodiment may be made to allow exposed metal **3702** in specific areas to optimize the cutting performance of the tool and minimize the required power input is shown, which may use a thinnest possible insulation in order to ensure better tissue contact with the exposed metal surface. The sheath **3402** with no exposed metal using insulation **3704** is also provided. In this embodiment, the cutting element, or the cutting arm, is not used. Rather, it may use RF power for cutting.

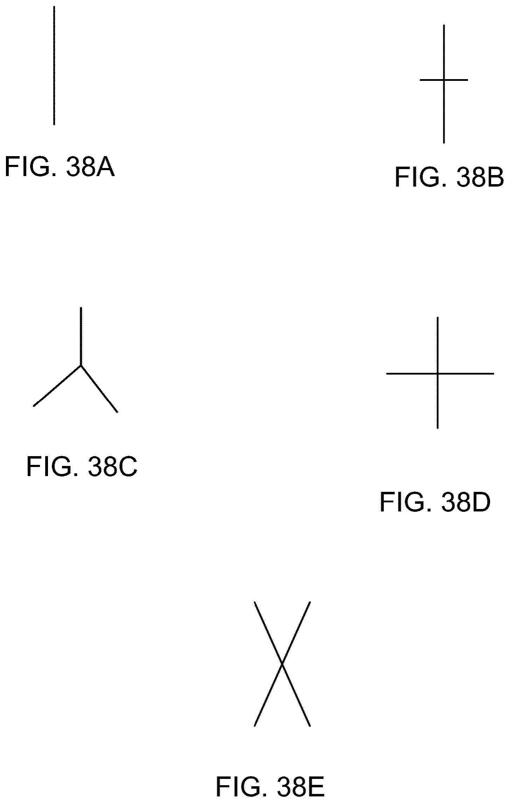

**FIGS. 38A-E**are schematics showing how the cutting implements may be used to optimize cutting performance and minimize power input in accordance with one aspect of the present disclosure. Various cutting shapes are shown that may be created by the embodiments through the interatrial septum. These shapes may be created with multiple cutting arms or using a single cutting arm that may be rotated and used multiple times.

**FIGS. 39-53** describe a third embodiment with delivery sheaths and a helical anchor for tying tissue together. Components described below may be placed into a catheter shaft. The shaft internally may have a number of different lumens and channels for these components. In one embodiment, separate tools may be used for each of the components. These tools may follow along the guidewire that is in place. Techniques and/or devices are shown below to provide large bore transseptal access with subsequent atrial re-access.

Turning to **FIG. 39****,** an isometric view of exemplary tissue **900** being crossed using an illustrative guidewire **102** in accordance with one aspect of the present disclosure is provided. The technique may initially begin with the guidewire **102** piercing or crossing into the tissue **900.** The guidewire **102** may be brought in through the vascular introduction sheath which may be routed cranially up the inferior vena cava to the right atrium. The guidewire **102** may be placed to direct therapeutic or diagnostic catheters into a region of the heart.

**FIG. 40** is an isometric view of suture anchors **4002** in delivery sheaths **4004** in accordance with one aspect of the present disclosure. The delivery sheaths **4004** may house other components in addition to the tissue anchors **4002.** Using the guidewire, the delivery sheaths **4004** may be directed towards the tissue **900** of the atrial septum.

**FIG. 41** is an isometric view of illustrative suture anchors **4002** in delivery sheaths **4004** about to penetrate the tissue **900** in accordance with one aspect of the present disclosure. The suture anchors **4002** may be placed at a first puncture point **4102** and a second puncture point **4104** which may be directed through a guidewire **102.** Thereafter, they may be inserted into the tissue **900.**

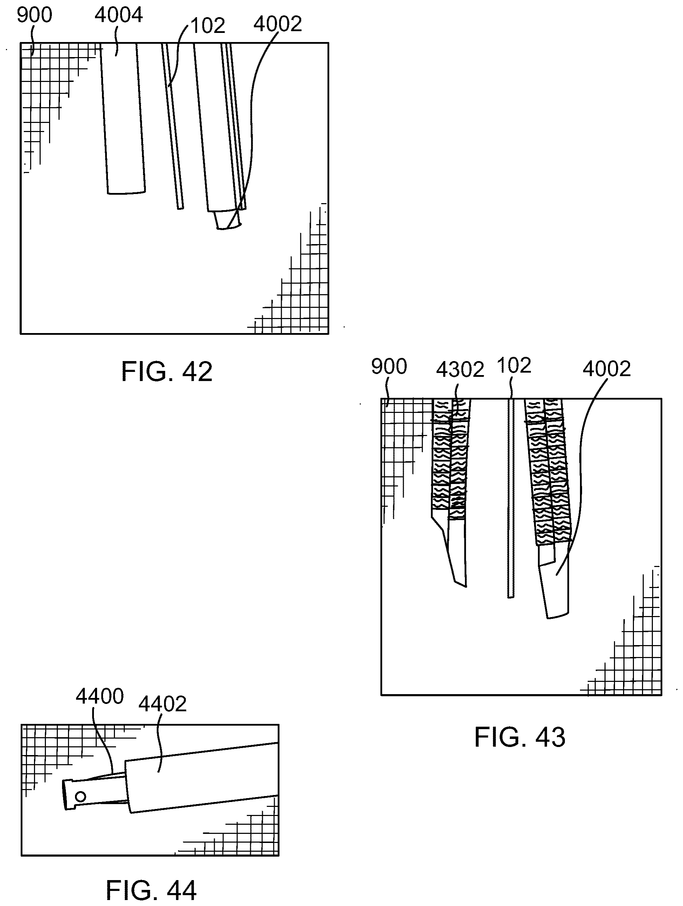

Referring to **FIG. 42****,** an isometric view of the illustrative suture anchors **4002** in delivery sheaths **4004** engaging or penetrating the tissue **900** in accordance with one aspect of the present disclosure is provided. The suture anchors **4002** in delivery sheaths **4004** may engage or penetrate the tissue **900** near the guidewire **102.**

**FIG. 43** is an isometric view of the illustrative suture anchors **4002** in delivery sheaths **4004** engaging the tissue **900** with suture control lines **4302** attached in accordance with one aspect of the present disclosure. The suture control lines **4302** may be used for holding sutures in place within the tissue **900** near the guidewire **102** while operations are being performed. The delivery sheaths have been removed to expose the suture control lines **4302.**

**FIG. 44** is an isometric view of an illustrative cutting implement **4400** in a sheathed position in accordance with one aspect of the present disclosure. The cutting implement **4400** may be tucked away into the sheath **4402.** The cutting implement **4400** may be circuitous with a guidewire, that is, its guidance may be based on the guidewire to the tissue.

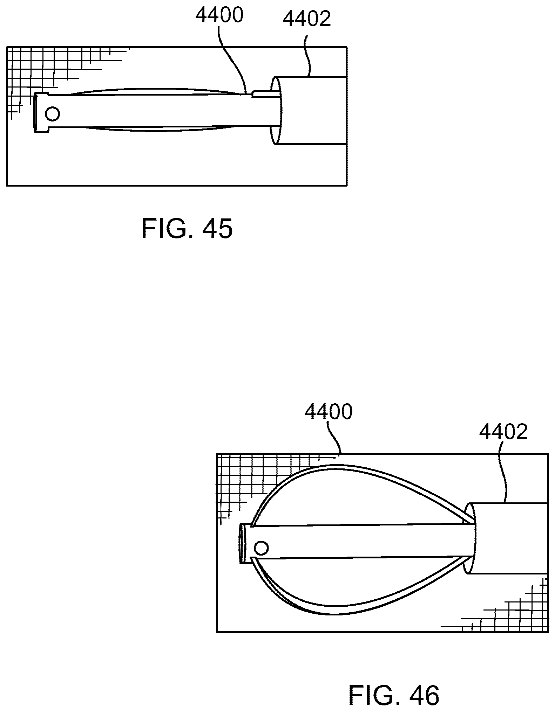

Turning to **FIG. 45****,** an isometric view of the illustrative cutting implement **4400** in an unsheathed position in accordance with one aspect of the present disclosure is provided. The cutting implement **4400** may be extended outside the sheath **4402.** This may be performed at a proximal end by an advancing mechanisms through the sheath **4402.**

**FIG. 46** is an isometric view of the illustrative cutting implement **4400** in an unsheathed position and expanded in accordance with one aspect of the present disclosure. The cutting implement **4400** may be spring loaded such that after extending outside the sheath **4402,** it may be expand symmetrically. The cutting implement **4400** may also be expanded through a pull-wire or other mechanism. Expanding the cutting implement **4400** may facilitate tissue incision.

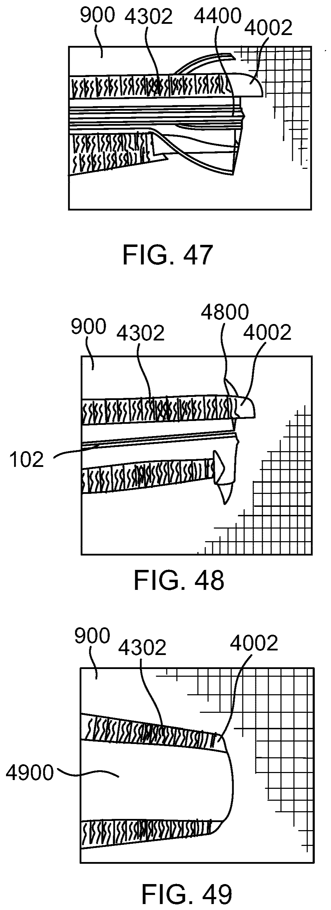

**FIG. 47** is an isometric view of the illustrative cutting implement **4400** making an incision or cut into the tissue **900** in accordance with one aspect of the present disclosure. The suture anchors **4002** in the delivery sheaths may be used to hold the tissue **900** in place while allowing guidance of the cutting implement **4400.** The cutting implement **4400** may make incisions between the two suture anchors **4002** in the delivery sheaths.

Referring to **FIG. 48****,** an isometric view of an illustrative incision **4800** in the tissue **900** with a guidewire **102** passing through it in accordance with one aspect of the present disclosure is provided. With the cutting implement removed, the two suture anchors **4002** coupled to the suture control lines **4302** along with the guidewire **102** remain.

**FIG. 49** is an isometric view of an advancing of an exemplary therapeutic instrument **4900** passing through the incision in the tissue **900** over the guidewire in accordance with one aspect of the present disclosure. The therapeutic instrumentation **4900** may be advanced through the guidewire and through the incision in the tissue **900.** The therapeutic instrumentation **4900** may be disposed between the two suture anchors **4002** coupled to the suture control lines **4302.**

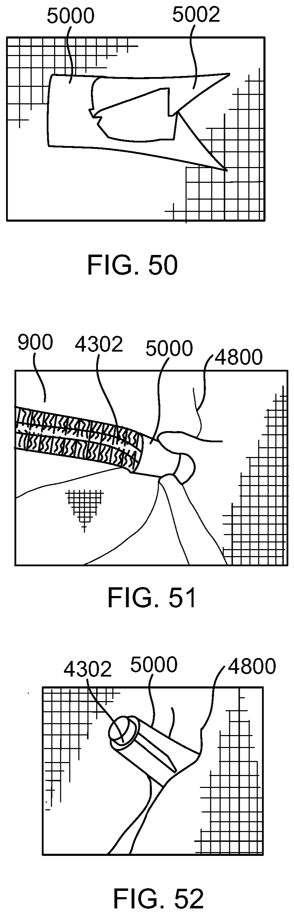

**FIG. 50** is an isometric view of an illustrative tissue anchor lock **5000** with helical barbs **5002** in accordance with one aspect of the present disclosure. The lock **5000** with helical barbs **5002** may be implanted into the tissue, which will be shown below, to close an incision.

Turning to **FIG. 51****,** an isometric view of the illustrative tissue anchor lock **5000** with helical barbs engaged in the tissue **900** passed over the suture control lines **4302** in accordance with one aspect of the present disclosure is provided. In this technique, the tissue **900,** having the incision **4800,** may be twisted with the tissue anchor lock **5000** using the helical barbs. This may secure the sutures, anchors and tissue **900** to one another.

**FIG. 52** is an isometric view of the illustrative tissue anchor lock **5000** with helical barbs engaged in the tissue passed over the suture control lines **4302** trimmed to the level of the anchor in accordance with one aspect of the present disclosure. The excess suture control lines **4302** may be trimmed through a separate cutting implement that is guided to the tissue using the guidewire. The incision **4800** may be closed with the lock **5000** which has minimal control lines **4302** attached thereto.

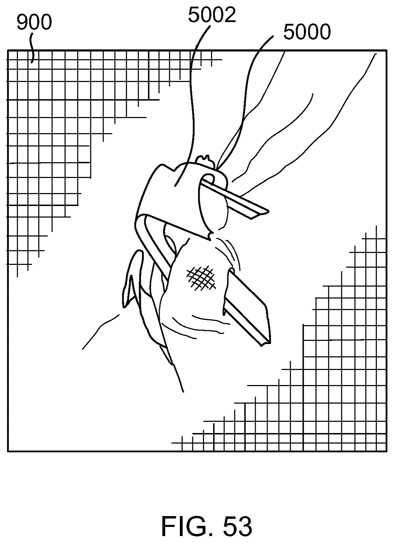

**FIG. 53** is an isometric view of the illustrative tissue anchor lock **5000** with helical barbs **5002** engaged in the tissue **900** from the other side of the tissue **900** in accordance with one aspect of the present disclosure. The lock **5000** may be interspersed between the tissue **900** to either heal with the tissue **900** and endothelize, or be absorbed or dissolve. The view is on an opposite side of atrial wall that was shown in **FIG. 52****.** The lock **5000** may grab or hook tissue **900** and then be twisted to seal the incision.

Multiple techniques were described above for closing an incision and allowing re-access. In addition, **FIGS. 54-69** provide a fourth embodiment localizing a plurality of pledgets made of biodegradable materials secured together with a knot to close the incision while allowing re-access at a later time. In the following, two pledgets may be used, however, fewer or more may be passed into the tissue for securing the area. The pledgets may be constructed from a bioabsorbable, biodegradable material including but not limited to; sugars, salts, collagen, PLGA, PLLA, other absorbable polymers, magnesium, and or other materials. The pledgets may be constructed from a combination of materials to facilitate different structural properties. Some of these materials may be traditional implant materials such as metals or polymers including, but not limited to, stainless steel, nitinol, cobalt chrome, PEEK, HDPE, and others.

Turning to **FIG. 54****,** an isometric view of an illustrative pledget **5402** made from biocompatible or bio-absorbable material in accordance with one aspect of the present disclosure is provided. The pledget **5402,** made from biocompatible or bioabsorbable material, may have a control line **5404** interlaced throughout. The pledget **5402** may be a single piece of material that is elongated with the control lines **5404** dispersed between the pledget **5402** and tied or fastened at the end of the pledget **5402.** The control lines **5404** may be interspersed between apertures within the pledget **5402.**

**FIG. 55** is an isometric view of an exemplary cannula **5500** piecing heart tissue **900** in accordance with one aspect of the present disclosure. The cannula **5500** may have a sharp distal end, but preferably uses RF energy to pierce the tissue **900.** The cannula **5500** may use RF at a distal end with insulation covering the tubular structure. The tubular structure may allow a pledget, or other mechanism, to be distributed therethrough. The cannula **5500** may be inserted into a vein, such as the femoral vein, to reach the patient's heart. The cannula **5500** may be set firmly in place. Different variations of cannulas **500** exist and the technique described herein is not limited to the cannula **500** shown.

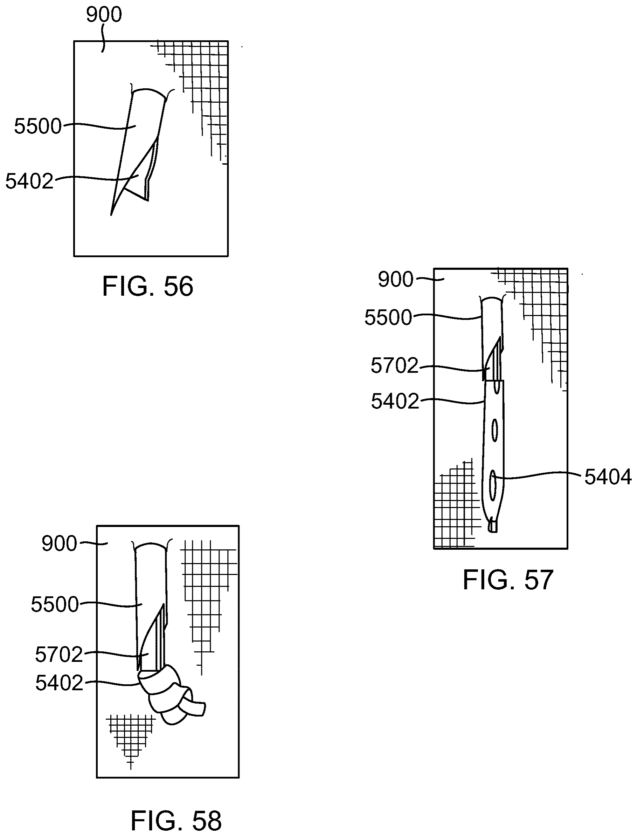

**FIG. 56** is an isometric view of the exemplary cannula **5500** piecing heart tissue **900** and the illustrative pledget **5402** made from biocompatible or bioabsorbable material advanced through the cannula **5500** in accordance with one aspect of the present disclosure. The cannula **5500** may pierce the tissue **900** with a circular cut. Typically, the cut may be small such that the pledget when compacted may plug or fill the cut, providing sufficient apposition to then tension the tissue for later closure. Other shapes for the cut may be used depending on the cross-section of the cannula **5500.**

Referring to **FIG. 57****,** an isometric view of the exemplary cannula **5500** piecing heart tissue **900** and the illustrative pledget **5402** made from biocompatible or bioabsorbable material advanced out of the cannula **5500** in accordance with one aspect of the present disclosure is provided. An advancing member **5702** may be placed into the cannula **5500** from a proximal end to force the pledget **5402** with control lines **5404** into the left atrium. In one embodiment, a proximal end of the pledget **5402** is coupled to the distal end of the advancing member **5702** through the control lines **5404** for activation of the pledget by shortening the distance and expanding the cross sectional area of the pledget .

**FIG. 58** is an isometric view of the exemplary cannula **5500** piecing heart tissue **900** and the illustrative pledget **5402** made from biocompatible or bioabsorbable material advanced out of the cannula **550** and a pull member tensioned to shorten the pledget **5402** in accordance with one aspect of the present disclosure. The control line **5404** may be pulled through the advancing member **5702.** The control line **5404,** which was interspersed between apertures within the pledget **5402,** may then cause the pledget **5402** to be retracted or shortened. This may cause the pledget **5402** to be bunched up.

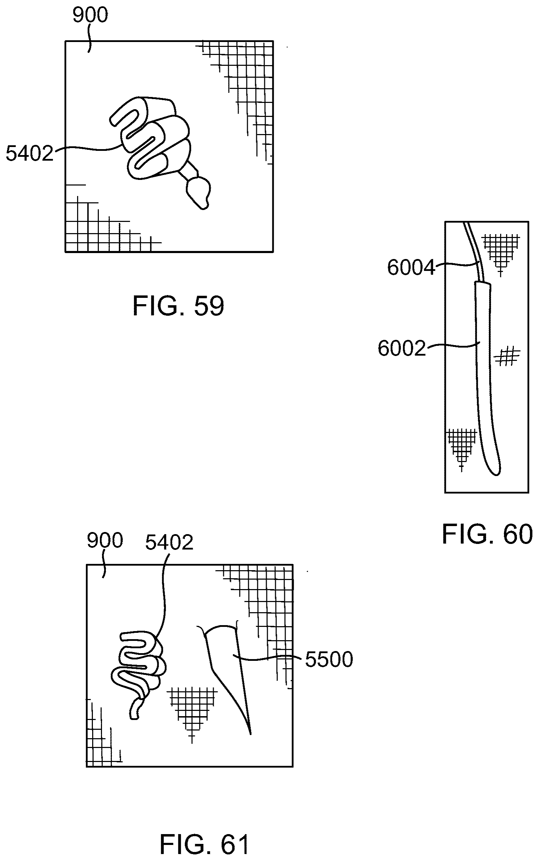

**FIG. 59** is an isometric view of the illustrative pledget **5402** made from biocompatible or bioabsorbable material tensioned to shorten the pledget **5402** with the exemplary cannula piecing heart tissue **900** withdrawn and retained on a heart tissue surface in accordance with one aspect of the present disclosure. With the cannula withdrawn and the pledget **5402** shortened and compacted, the initial cut made by the cannula has been plugged or secured.

Referring to **FIG. 60****,** an isometric view of an illustrative concentric pledget **6002** made from biocompatible or bioabsorbable material in accordance with one aspect of the present disclosure is provided. The concentric pledget **6002,** made from biocompatible or bioabsorbable material, may be introduced in a similar manner as the other pledget. The concentric pledget **6002** may be made of the same or similar materials as the other pledget. A control line **6004** may be coupled to the concentric pledget **6002** allowing the pledget **6002** to be pulled back such that the concentric pledget **6002** may become contracted or bundled expanding the cross sectional area to provide for greater apposition force to the tissue plane.

**FIG. 61** is an isometric view of the exemplary cannula **5500** piecing heart tissue **900** next to the pledget **5402** in accordance with one aspect of the present disclosure. The cannula **5500** may be placed into the tissue **900** near the other pledget **5402.** Typically, and as described earlier, the pledgets may be inserted by one another to maintain the integrity of the surrounding tissue as well as allow re-access. The placement may provide for a cutting implement to be disposed between the pledgets to make an incision as well as for a therapeutic or diagnostic instrument to be inserted into the incision.

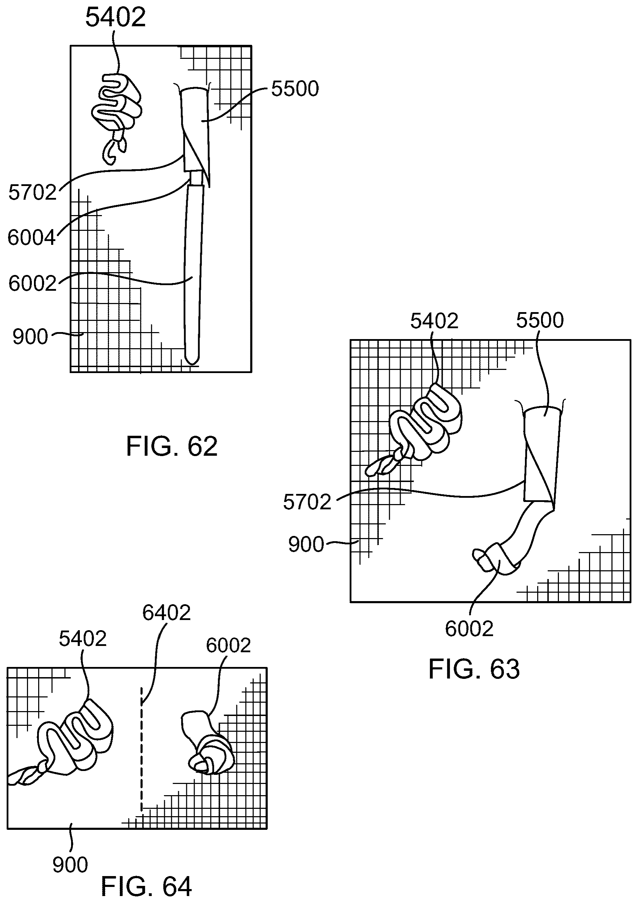

**FIG. 62** is an isometric view of the exemplary cannula **5500** piecing heart tissue **900** and the illustrative concentric pledget **6002** made from biocompatible or bioabsorbable material advanced out of the cannula **5500** in accordance with one aspect of the present disclosure. The advancing member **5702** may be used to push or advance the pledget **6002** through the cannula **5500.** The pledget **6002** connected to the control line **6004** may be pushed through the tissue **900** via the cannula **5500** next to the other pledget **5402.**

**FIG. 63** is an isometric view of the exemplary cannula **5500** piecing heart tissue **900** and the illustrative concentric pledget **6002** made from biocompatible or bioabsorbable material advanced out of the cannula **5500** and a pull member tensioned to shorten the pledget in accordance with one aspect of the present disclosure. The control line of the concentric pledget **6002** may be pulled through the advancing member **5702.** By doing so, the concentric pledget **6002** may be shorted or bundled.

Referring to **FIG. 64****,** an isometric view of the illustrative concentric pledget **6002** made from biocompatible or bioabsorbable material tensioned to shorten the pledget **6002** and an exemplary incision **6402** made between the pledgets **5402** and **6002** in accordance with one aspect of the present disclosure is provided. The pledgets **5402** and **6002** may provide anchor points where the tissue **900** may be tied together.

A cutting implement, as previously described, may be used to make the incision **6402** between them. While a straight cut is shown, other types of incisions **6402** may be made. These may include, but are not limited to, a straight cut, v cut, zig zag, or crescent arc. With both tissue securing pledgets **5402** and **6002** in place, the incision **6402** may then be made.

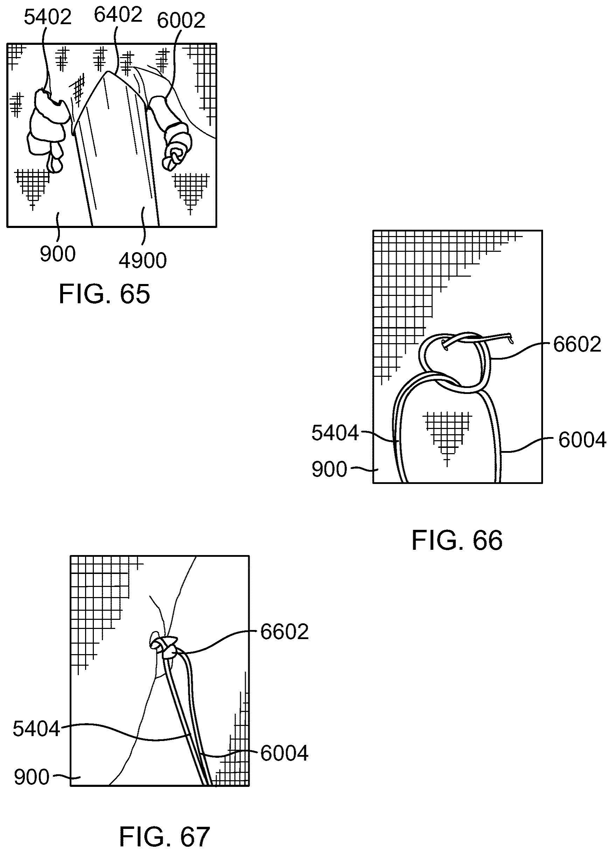

**FIG. 65** is an isometric view of the exemplary therapeutic instrument **4900** placed into the incision **6402** between the pledgets **5402** and **6002** into the tissue **900** in accordance with one aspect of the present disclosure. Widening of the incision **6402** may occur when the therapeutic instrument **4900** is placed therein. The therapeutic instrument **4900** may follow a guidewire that was inserted for the other mechanisms brought to the right atrium. A diagnostic instrument may also be used.

**FIG. 66** is an isometric view of an illustrative knot **6602** advanced up to the heart tissue **900** with the two pledget control lines **5404** and **6004** in accordance with one aspect of the present disclosure. Upon use and removal of the therapeutic instrument **4900,** the securing knot **6602** may be advanced through tightening of the control lines **5404** and **6004** of the pledgets.

Referring to **FIG. 67****,** an isometric view of the illustrative knot **6602** advanced up to the heart tissue **900** with the two pledget control lines **5404** and **6004** and tensioned tight from the knot side of the tissue **900** in accordance with one aspect of the present disclosure is provided. When pulled, the control lines **5404** and **6004** may advance the knot **6002** further to the tissue **900.** The control lines **5404** and **6004** may be cut to shorten them, which will be shown below.

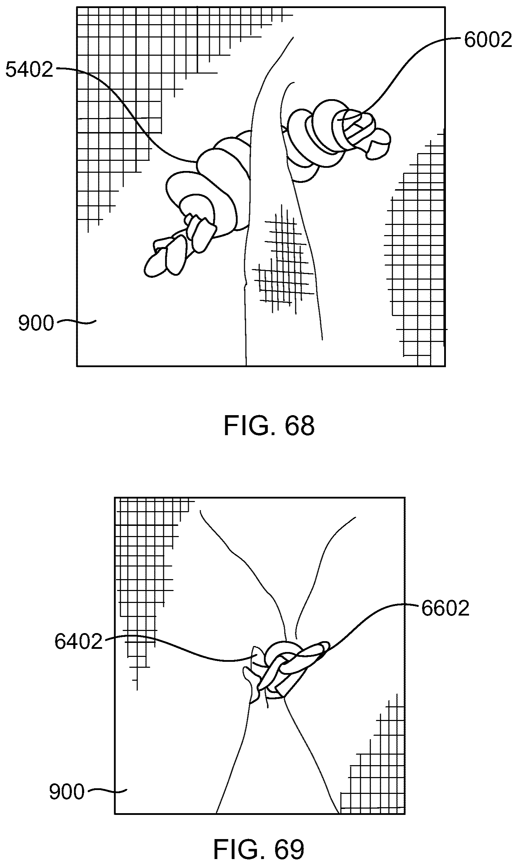

**FIG. 68** is an isometric view of the illustrative knot advanced up to the heart tissue **900** with the two pledget tension lines tightened from the pledget side of the tissue **900** in accordance with one aspect of the present disclosure. When tightened, the pledgets **5402** and **6002** may collapse towards one another with tissue **900** folded therebetween.

**FIG. 69** is an isometric view of the illustrative incision **6402** closed between the pledgets in accordance with one aspect of the present disclosure. Control lines used to make the knot **6602** may be removed or cut. This may remove any interference that they have within the patient.

In addition to suture and mechanical apparatuses to join the tissue edges together, adhesive materials may be used to seal or join the tissue either as a primary or supplementary or adjunct mechanism. These material may include, but are not limited to, adhesive cyanoacrylates, methoxypropyl cyanoacrylates, alkyl cyanoacrylates such as n-butyl, isobutyl or n-octyl cyanoacrylates, octylcyanoacrylate, butylcyanoacrylate, BioGlue^{®} Surgical Adhesive (BioGlue), bovine serum albumin (BSA), glutaraldehyde of purified (BSA), extracellular matrix ECM human connective tissue, autologous and homologous fibrin sealants, fibrin glue, Polyethylene glycol (PEG)-Based Hydrogel Sealants, hydrogel, methacryloyl-substituted tropoelastin (MeTro), and many others. These sealants may be biocompatible and resorbable.

In one embodiment, a 'bipolar' catheter type mechanism may be used to seal the tissue back together. For example, bipolar coagulating forceps used to stop a bleeding vessel may be used. There may be procedural order options to accomplish this procedure.

**FIG. 70** is an illustrative flow chart showing exemplary processes for allowing a large bore transseptal access with subsequent atrial re-access in accordance with one aspect of the present disclosure. These processes are for illustrative purposes and may be modified according to those techniques described herein. The processes may begin at **block 7000.**

At **block 7002,** a guidewire may be inserted into the heart chamber and distributed at the atrial septum. The guidewire may be inserted into the venous circulatory system through the vascular introduction sheath. The initial percutaneous puncture or incision, by way of example, may be at the patient's femoral vein. Other areas where the guidewire may enter into the patient may include, but is not limited to, a jugular vein, subclavian artery, subclavian vein, or brachial artery and vein.

The guidewire may be routed cranially up the inferior vena cava to the right atrium of the heart. The guidewire may be placed at the atrial septum so that it is used to direct therapeutic or diagnostic catheters into a region of the heart. In turn, the guidewire may be temporarily or removably fixed at the atrial septum.

At **block 7004,** a puncture may be made by a needle through the atrial septum into the left atrium. The septal penetrator may be a needle or axially elongate structure with a sharp, pointed distal end. In one embodiment, the septal penetrator may be resident within the guidewire. The septal penetrator may be actuated at the proximal end of the vascular device through a control mechanism such as a button, lever, handle, or trigger which may be affixed, permanently or removably by way of a linkage, pusher rod, electrical bus, or the like that runs the length of the device.

The guidewire may be used as the puncture device. The guidewire may have a tip that facilitates the crossing of the septum such as, but not limited to, a sharp end, a helical end, a RF energy electrode tip, other energy tip, or other device to aid the tissue penetration.

Sutures may be pulled through the punctures at **block 7006** through an anchor. The sutures may be bundled within a suture bundle and stored in the recess of the anchor. The catheter shaft may be positioned in the right atrium with the anchor, having the suture bundle, advanced into the left atrium through the puncture. In turn, the suture bundle may be unspooled by snares which capture needles coupled to the end of the sutures. The snares with the needles may be pulled into the catheter shaft. The sutures from the suture bundles may be managed through the snares.

Sutures may be placed from the right to left atrium or from the left to right atrium depending on the apparatus. The suture may be placed repeatedly across the septum for multiple points of engagement. The sutures may be another type of apparatus such as a helical anchor or barb device. The placement of the sutures may also be provided after **block 7012,** when the therapy is complete.

At **block 7008,** and after the sutures are in place, a cut may be made at the interatrial septum using the cutting implement proximal to the needle passing. The sutures may be deployed through holes made near the initial puncture. Cuts or incisions that are made may be parallel such that the sutures are not cut by the cutting implement. The cutting implement may be coupled to the catheter to make sure that the cutter does not accidentally cut the sutures.

Various cutting implements were described herein. Mechanical or RF energy may be used. When using electrical energy, the amount of exposed metal may be minimized through insulation such that the exposed metal may only exist in the desired tissue cutting region of the tool. Mechanical energy may use blades that may be deployed in the singular direction or may be symmetrical. The cutting may be performed from the right to left atrium or from the left to right depending on the apparatus. The cutting may be post anchor placement. Alternatively, the cutting may precede the suture anchor placement, described at **block 7006.** The cutting may be integrated into **blocks 7002** and **7004** above with a device that punctures and cuts the tissue.

At **block 7010,** the sutures may be managed. That is, the sutures may be pulled towards the vessel wall and be shifted or manipulated such that they do not interfere or entangle with the therapy instrumentation catheters. The tissue suture management lines may be managed in a lumen of an access sheath or in a separate catheter.

Therapy may be performed using the therapeutic instrument at **block 7012.** The therapeutic instrument may perform diagnosis and therapeutic intervention to correct atrial fibrillation, perform mitral valve repair, correct septal defects, perform implantation of a cardiac prosthesis, or the like. The therapy or diagnosis may be performed in the left atrium. In turn, the therapeutic instrument may be removed.

At **block 7014,** the control sutures may be used to close the incision. The amount of this closure may be adjusted to meet the desired therapeutic goals of the procedure. It may be desirable to completely seal the incision, or leave a passage for relief of excess pressure from one side to the other. In one example, the control sutures may be pressed against the tissue which was described above. The processes may end at **block 7016.**

Other techniques may be used for allowing large bore transseptal access with subsequent atrial re-access. For example, and in this embodiment, the method may include puncturing the septum for needle passing, leaving sutures or some other anchor behind, and then performing a procedure. The anchors or sutures left behind may be cinched together to close the septum. Excess sutures may be then be cut.

In another technique for atrial re-access, the septum may be cut first. The septum may be stabilized through a needle puncture radius so that the needles may pass through the previous cut septum. The needle passing device may be introduced. Needles on the left atrium side may then be snared/grabbed and pulled through the catheter. In turn, pre-shaped nitinol wire may be used to loop through the septum. Control structures may be used to close the incision with the excess sutures cut.

In yet another technique, the transseptal access may be obtained via a standard transseptal approach. The guidewire may be left across the transseptal access site in the left atrium. The vascular device for slicing, enlarging and placing sutures is advanced over the guidewire. Four cutting members radially spaced may slice the septum and enlarge the transseptal access point in a controlled and consistent manner. Needles may then puncture the septum and pass a suture through the interatrial tissue in a location between the slices such that when cinched together they most optimally close the iatrogenic ASD. The vascular device may then be removed, leaving the four sutures in place across the septum. The sutures may be pulled out of the vein and remain temporarily in place in the inferior vena cava vein until later in the procedure.

In yet another technique the mechanism to facilitate the delivery of the suture, anchors, cutting implements, and closing features may be integrated into the therapeutic instrument to minimize the exchange of devices in the patient.

In yet another technique, no foreign body may be left behind. The technique, with associated device or devices, may come into the atrium after the procedure and cinch the tissue to be apposed for a time to promote healing while sealing them. In turn, the technique may include removing the structure or apparatus. This technique may be the combination of a controlled incision and then later cinching with an apparatus described herein, or that is known in the art. The apparatus may include, but is not limited to, grabbers, forceps, helical anchors, pinchers, knots, suction devices, barbs, or the like. This technique may then promote the tissue to heal by itself due to cut morphology. The time of this temporary apposition may range from minutes to days, or weeks, depending on the amount of tissue healing desired.

In some embodiments, the anchors described above may subsequently be removed from the tissue, or alternatively may be left in place. The techniques or procedures for removing the anchors may use grabbers, snares, cutting elements, or engagement features specific to the mechanism left in place. A mechanical feature may be added on the right atrial side of the anchoring device such that it may be subsequently grabbed and unscrewed. This mechanical feature may be in the shape of a hook, an oval, or the like. This feature may protrude off of the right atrial septum such that it may be grasped with a snare and rotated out of the tissue, for example.

The foregoing description is provided to enable any person skilled in the relevant art to practice the various embodiments described herein. Various modifications to these embodiments will be readily apparent to those skilled in the relevant art and generic principles defined herein may be applied to other embodiments. Thus, the claims are not intended to be limited to the embodiments shown and described herein, but are to be accorded the full scope consistent with the language of the claims, wherein reference to an element in the singular is not intended to mean "one and only one" unless specifically stated, but rather "one or more." All structural and functional equivalents to the elements of the various embodiments described throughout this disclosure that are known or later come to be known to those of ordinary skill in the relevant art are expressly incorporated herein by reference and intended to be encompassed by the claims. Moreover, nothing disclosed herein is intended to be dedicated to the public regardless of whether such disclosure is explicitly recited in the claims.

The following numbered examples of combinations of features are presented by way of technical explanation and do not limit the scope of the invention as defined by the claims.
Example 1. A vascular device for performing a transseptal puncture, comprising:
   a body;
   an anchor extending from a distal end of the body through a shaft disposed within the body;
   at least one suture coupled to at least one needle within the anchor;
   at least one catch extending from the body to pull the at least one needle into the body for placing the at least one suture; and
   a cutting implement between the body and anchor coupled to an actuating shaft aligned with the at least one suture.
2. The vascular device for performing the transseptal puncture of example 1, comprising a guidewire disposed within the body and anchor.
3. The vascular device for performing the transseptal puncture of example 1, wherein a face of the body and anchor are angled.
4. The vascular device for performing the transseptal puncture of example 1, wherein the body comprises a rectangular-shaped lumen for the shaft of the anchor.
5. The vascular device for performing the transseptal puncture of example 1, wherein the body comprises at least one lumen for the at least one catch.
6. The vascular device for performing the transseptal puncture of example 1, wherein the at least one suture is stored in a recess channel of the anchor.
7. The vascular device for performing the transseptal puncture of example 6, wherein the at least one suture comprises two ends coupled to two needles extending towards the body.
8. The vascular device for performing the transseptal puncture of example 6, wherein the recess channel releases the at least one suture after pulled by the at least one catch.
9. The vascular device for performing the transseptal puncture of example 1, wherein the device comprises two sutures with each suture coupled to two needles.
10. The vascular device for performing the transseptal puncture of example 1, wherein the at least one catch is pulled from a proximal end of the body.
11. The vascular device for performing the transseptal puncture of example 1, wherein the actuating shaft of the cutting implement telescopes over the shaft of the anchor.
12. The vascular device for performing the transseptal puncture of example 1, wherein the cutting implement is expanded radially from the body through the actuating shaft.
13. The vascular device for performing the transseptal puncture of example 12, wherein the actuating shaft causes the cutting implement to bow at a greater length than a diameter of the body.
14. The vascular device for performing the transseptal puncture of example 12, wherein the actuating shaft causes a linkage system to expand the cutting implement.
15. The vascular device for performing the transseptal puncture of example 1, wherein the cutting implement uses mechanical or electrical energy.
16. The vascular device for performing the transseptal puncture of example 15, wherein the mechanical or electrical energy comes from at least one of a blade, ceramic, electrocautery technique, radio frequency, plasmajet vaporization, ultra-sonic, high voltage vaporization, controlled dilation, heat and cold.
17. A septal orifice closure apparatus allowing re-access, comprising:
   a body on a first side of a septal orifice in a septum of a heart;
   an anchor on a second side of the septal orifice extending from a distal end of the body through a shaft disposed within the body;
   at least one suture coupled to at least one needle disposed within the anchor;
   at least one catch extending from the body to pull the at least one needle into the body for placing the at least one suture; and
   a cutting implement between the body and anchor coupled to an actuating shaft aligned with the at least one suture.
18. The septal orifice closure apparatus of example 17, wherein the cutting implement uses mechanical or electrical energy.
19. The septal orifice closure apparatus of example 17, comprising a guidewire disposed within the body.
20. The septal orifice closure apparatus of example 17, wherein the cutting implement comprises a first circuit for a first puncture and a second circuit for a larger cut.
21. The septal orifice closure apparatus of example 17, wherein the cutting implement comprises a pull ring or spring to open and close the cutting implement.
22. A vascular closure apparatus comprising:
   an anchor positioned through a puncture in a vessel wall and operable between retracted and expanded positions from a body;
   at least one suture disposed within the anchor;
   at least one needle coupled to the at least one suture extending through the vessel wall adjacent to the puncture to connect the at least one suture when the anchor is in the expanded position;
   at least one catch extending from the body to pull the at least one needle into the body for placing the at least one suture; and
   a cutting implement between the body and anchor coupled to an actuating shaft aligned with the at least one suture.
23. The vascular closure apparatus of example 22, wherein the anchor comprises a recess channel for storing the at least one suture.
24. The vascular closure apparatus of example 23, wherein the recess is shaped permitting unspooling of the at least one suture once pulled through the at least one catch.
25. The vascular closure apparatus of example 22, wherein the anchor is operable between the retracted and expanded positions to capture the vessel wall between the anchor and the body.
26. The vascular closure apparatus of example 22, wherein the at least one needle extends radially outward from the anchor towards the body.
27. The vascular closure apparatus of example 22, wherein the body comprises a lumen for the at least one catch.
28. The vascular closure apparatus of example 22, wherein the body comprises four lumens for four catches hooking into four needles of two sutures.
29. The vascular closure apparatus of example 22, wherein the cutting implement is expanded radially from the body through the actuating shaft.
30. The vascular closure apparatus of example 29, wherein the cutting implement expanded radially from the body is parallel to the at least one suture.
31. A method of performing a septal crossing in a vessel wall, comprising:
   providing a delivery catheter having a body and an anchor;
   inserting the anchor through a puncture in the vessel wall;
   operating the anchor into an expanded position capturing the vessel wall between the body and the anchor to expose at least one needle;
   capturing the at least one needle through the vessel wall adjacent to the puncture and into engagement with at least one suture; and
   positioning the at least one suture in the vessel wall.
32. The method of performing the septal crossing in the vessel wall of example 31, comprising placing a guidewire to the vessel wall and extending the delivery catheter over the guidewire.
33. The method of performing the septal crossing in the vessel wall of example 31, wherein providing the delivery catheter comprises transferring the delivery catheter in a retracted position to the vessel wall.
34. The method of performing the septal crossing in the vessel wall of example 31, wherein capturing the at least one needle through the vessel wall comprises unspooling the at least one suture within the anchor.
35. The method of performing the septal crossing in the vessel wall of example 31, comprising extending a cutting implement between the body and anchor through an actuating shaft aligned with the at least one suture.
36. The method of performing the septal crossing in the vessel wall of example 35, comprising telescoping the actuating shaft over a shaft connecting the body and anchor of the delivery catheter.
37. The method of performing the septal crossing in the vessel wall of example 35, comprising radially expanding the cutting implement through a linkage system in line with the actuating shaft.
38. The method of performing the septal crossing in the vessel wall of example 35, wherein the cutting implement is parallel to the at least one suture.
39. The method of performing the septal crossing in the vessel wall of example 31, comprising removing the delivery catheter.
40. The method of performing the septal crossing in the vessel wall of example 39, comprising providing a therapy catheter gaining access to the vessel wall.
41. The method of performing the septal crossing in the vessel wall of example 40, comprising:
   knotting the at least one suture; and
   pushing the at least one suture towards the vessel wall creating a closing force.
42. The method of performing the septal crossing in the vessel wall of example 41, comprising removing excess within the at least one suture.
43. The method of performing the septal crossing in the vessel wall of example 31, wherein capturing the at least one needle through the vessel wall comprises advancing two needles tied to opposite sides of a suture.
44. The method of performing the septal crossing in the vessel wall of example 43, wherein advancing the two needles tied to opposite sides of a suture is performed concurrently.
45. The method of performing the septal crossing in the vessel wall of example 31, comprising promoting tissue edge apposition through a helical anchor.
46. A vascular apparatus comprising:
   a delivery system having at least one anchor penetrating a tissue plane, the at least one anchor having a suture;
   a cutting implement positioned into the tissue plane facilitating an incision;
   a therapeutic instrument advanced into the incision; and
   a fastener securing the suture with tissue of the tissue plane.
47. The vascular closure system of example 46, comprising a guidewire controlling the delivery system, cutting implement, therapeutic instrument and fastener to the tissue plane.
48. The vascular closure system of example 46, wherein the suture is controlled by a control line.
49. The vascular closure system of example 46, wherein the at least one anchor is within a delivery sheath.
50. A septal orifice closure apparatus comprising
   a first pledget introduced into a tissue plane through a cannula, wherein the first pledget is coupled to a control line tensioning the first pledget after introduction into the tissue plane;
   a second pledget introduced into the tissue plane through the cannula, wherein the second pledget is coupled to a control line tensioning the second pledget after introduction into the tissue plane;
   a cutting implement making an incision between the first pledget and second pledget;
   a therapy device passed through the incision; and
   a knot made of the control line of the first pledget and the control line of second pledget tensioning the first pledget and second pledget with tissue from the tissue plane therebetween.
51. The septal orifice closure apparatus of example 50, comprising an advancing member within the cannula pushing the first pledget and the second pledget through the tissue plane.
52. A device for puncturing an atrial septum of a patient, comprising:
   a body;
   a tip extending from a distal end of the body; and
   a cutting member in a collapsed state disposed between the body and tip, wherein the tip followed by the cutting member penetrates into a tissue plane, the cutting member expanded after passing through the tissue plane.
53. The device for puncturing the atrial septum of the patient of example 52, wherein the cutting member is concealed within the body before penetrating through the tissue plane.
54. The device for puncturing the atrial septum of the patient of example 52, wherein the tip comprises RF energy or pressure.
55. The device for puncturing the atrial septum of the patient of example 52, wherein the cutting member comprises four equidistant blades extending radially from the device when expanded.
56. The device for puncturing the atrial septum of the patient of example 52, comprising a visual tool confirming a position of the device.
57. The device for puncturing the atrial septum of the patient of example 52, comprising a plurality of needles with anchors.
58. A vascular apparatus comprising:
   a delivery system;
   a tip extending from a distal end of the delivery system; and
   a cutting implement disposed between the delivery system and tip.
59. The vascular apparatus of example 58, wherein the cutting implement comprises electrical insulation selectively removed.
60. The vascular apparatus of example 58, wherein the cutting implement comprises an atraumatic tip.
61. The vascular apparatus of example 60, wherein the cutting implement comprises cutting surfaces on both sides of the atraumatic tip.
62. The vascular apparatus of example 58, wherein the cutting implement comprises a cutting arm expanding radially through a slit in a needle type sheath.
63. The vascular apparatus of example 62, wherein the cutting arm is adjustable through a pull-wire or spring.
64. The vascular apparatus of example 58, wherein the tip is entirely insulated except at a distal region.
65. The vascular apparatus of example 58, wherein the tip and cutting implement are on separate parallel circuits.
66. The vascular apparatus of example 58, wherein the cutting implement comprises a plurality of cutting surfaces energized through different switches.
67. A method of instrumenting the left atrium comprising:
   puncturing a septum with a needle;
   placing at least one suture behind the septum;
   advancing a therapeutic instrument into the puncture; and
   cinching the at least one suture closing the puncture.
68. The method of instrumenting the left atrium of example 67, comprising cutting an excess from the at least one suture.
69. The method of instrumenting the left atrium of example 67, comprising accessing a femoral vein to access a right atrium for puncturing the septum.
70. A method of closing a septal orifice comprising:
   creating a transseptal access through a wire;
   inserting a delivery catheter over the wire;
   enlarging the transseptal access through a cutting implement of the delivery catheter;
   inserting at least one suture coupled to a needle that passes around the transseptal access;
   cinching the transseptal access with the at least one suture; and
   removing the delivery catheter.
71. The method of closing the septal orifice of example 70, wherein the cinching is close to an iatrogenic atrial septal defect.
72. The method of closing the septal orifice of example 70, wherein enlarging the transseptal access through the cutting implement comprises radially expanding four cutting members of the cutting implement.
73. The method of closing the septal orifice of example 70, wherein enlarging the transseptal access through the cutting implement comprises determining a user-selected cut length.
74. The method of closing the septal orifice of example 70, wherein enlarging the transseptal access through the cutting implement comprises making a user-selected linear slit.
75. The method of closing the septal orifice of example 70, wherein enlarging the transseptal access through the cutting implement comprises making a user-selected non-linear slit.

## Claims

1. A vascular device for performing a transseptal puncture, comprising:
a catheter shaft (702);
an anchor (704) located at and extendable away from a distal end of the catheter shaft (704);
one or more needles (804) extending rearward from the anchor (704) towards the catheter shaft (702);
at least one suture (1102, 1302) coupled to one of the needles (804);
one or more needle lumens (1002) within the catheter shaft (702) that are each axially aligned with one of the one or more needles;
a cutting implement (1500) between the catheter shaft (702) and anchor (704); the cutting implement (1500) having an expanded state with a diameter larger than the catheter shaft (702).

2. The vascular device for performing the transseptal puncture of claim 1, wherein the cutting implement (1500) is connected to an actuating shaft (1502) and wherein the anchor (704) is connected to an advancement shaft (802).

3. The vascular device for performing the transseptal puncture of claim 2, wherein the actuating shaft (1502) slides relative to the advancement shaft (802) in a telescoping arrangement.

4. The vascular device for performing the transseptal puncture of claim 1, wherein the cutting implement (1500) in the expanded state is parallel relative to the at least one suture (1102, 1302) to remove any potential to cut the at least one suture (1102, 1302).

5. The vascular device for performing the transseptal puncture of claim 1, wherein the cutting implement (1500) is a linkage system (1504) comprising blades.

6. The vascular device for performing the transseptal puncture of claim 1, wherein the at least one suture (1102, 1302) is stored in a recess channel (1104) of the anchor (704).

7. The vascular device for performing the transseptal puncture of claim 6, wherein the recess channel (1104) is configured to release the at least one suture after being pulled by a snare (1202).

8. The vascular device for performing the transseptal puncture of claim 6, wherein the recess channel (1104) contains two of the sutures (1102, 1302).

9. The vascular device for performing the transseptal puncture of claim 6, wherein the recess channel (1104) is shaped to release the sutures (1102, 1302).

10. The vascular device for performing the transseptal puncture of claim 6, wherein the recess channel (1104) contains two of the sutures (1102, 1302).

11. The vascular device for performing the transseptal puncture of claim 1, further comprising snares (1202) located within the needle lumens (1002).

12. The vascular device for performing the transseptal puncture of claim 11, wherein the needle lumens (1002) include recesses (1204) that snares (1202) engage with.

13. The vascular device for performing the transseptal puncture of claim 11, further comprising a mechanism accessible at a proximal end of the catheter shaft (702) to move the snares (1202) to catch and pull the needles (804).

14. The vascular device for performing the transseptal puncture of claim 1, wherein the one or more needles (804) comprise four needles and wherein the at least one suture (1102, 1302) comprises two sutures.

15. The vascular device for performing the transseptal puncture of claim 1, wherein the cutting implement is configured to use mechanical or electrical energy.
